(19) Europäisches Patentamt
European Patent Office
Office européen des brevets

(11) **EP 4 570 254 B1**

(12) # FASCICULE DE BREVET EUROPEEN

(45) Date de publication et mention de la délivrance du brevet:
**02.09.2026 Bulletin 2026/36**

(21) Numéro de dépôt: **24223544.8**

(22) Date de dépôt: **13.07.2020**

(51) Classification Internationale des Brevets (IPC):
***A61K 31/713*** *(2006.01)* ***C12N 15/113*** *(2010.01)*
***A61P 35/00*** *(2006.01)* ***A61P 29/00*** *(2006.01)*

(52) Classification Coopérative des Brevets (CPC):
**C12N 15/1137; A61K 31/713; A61P 29/00; A61P 35/00;** C12N 2310/14

(54) **SEQUENCES SIARN CIBLANT L'EXPRESSION DU GENE JAK1 HUMAIN POUR UNE UTILISATION THERAPEUTIQUE**

AUF DIE EXPRESSION DES MENSCHLICHEN JAK1 GENS GERICHTETE SIRNA-SEQUENZEN ZUR THERAPEUTISCHEN VERWENDUNG

SIRNA SEQUENCES TARGETING THE EXPRESSION OF THE HUMAN JAK1 GENE FOR THERAPEUTIC USE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.07.2019 FR 1907968**

(43) Date de publication de la demande:
**18.06.2025 Bulletin 2025/25**

(62) Numéro(s) de document de la (des) demande(s) initiale(s) en application de l'article 76 CBE:
**23197707.5 / 4 282 481**
**20740302.3 / 3 999 075**

(73) Titulaire: **Commissariat à l'Energie Atomique et aux Energies Alternatives**
**75015 Paris (FR)**

(72) Inventeurs:
- **CLEMENT, Flora**
  **38054 GRENOBLE (FR)**
- **SULPICE, Eric**
  **38054 GRENOBLE (FR)**
- **GIDROL, Xavier**
  **38054 GRENOBLE (FR)**

(74) Mandataire: **Lavoix**
**2, place d'Estienne d'Orves**
**75441 Paris Cedex 09 (FR)**

(56) Documents cités:
**EP-A1- 1 325 955**

- **CLÉMENT FLORA ET AL: "Therapeutic siRNAs Targeting the JAK/STAT Signalling Pathway in Inflammatory Bowel Diseases", vol. 16, no. 2, 23 February 2022 (2022-02-23), NL, pages 286 - 300, XP093095008, ISSN: 1873-9946, Retrieved from the Internet <URL:https://watermark.silverchair.com/jjab129.pdf?token=AQECAHi208BE49Ooan9kkhW_Ercy7Dm3ZL_9Cf3qfKAc485ysgAAA3EwggNtBgkqhkiG9w0BBwagggNeMIIDWgIBADCCA1MGCSqGSIb3DQEHATAeBglghkgBZQMEAS4wEQQMzPplhjFqc8Dzd5v-AgEQgIIDJOcNLurNKdUQWLZkhX0I5ldC-kCo6W-uY3Z3kLWapZm18WQ-vzUUPGyu0ONlf_-c_25CneMQfCL70podEnCuCZsz0i-P> DOI: 10.1093/ecco-jcc/jjab129**
- **DAN LIU ET AL: "Down-regulation of JAK1 by RNA interference inhibits growth of the lung cancer cell line A549 and interferes with the PI3K/mTOR pathway", JOURNAL OF CANCER RESEARCH AND CLINICAL ONCOLOGY, SPRINGER, BERLIN, DE, vol. 137, no. 11, 23 August 2011 (2011-08-23), pages 1629 - 1640, XP019964966, ISSN: 1432-1335, DOI: 10.1007/S00432-011-1037-6**
- **RUI LIXIN ET AL: "Epigenetic gene regulation by Janus kinase 1 in diffuse large B-cell lymphoma", PROCEEDINGS OF THE NATIONAL ACADEMY OF SCIENCES, vol. 113, no. 46, 31 October 2016 (2016-10-31), XP093095024, ISSN: 0027-8424, DOI: 10.1073/pnas.1610970113**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 4 570 254 B1

- **RUI LIXIN ET AL: "Epigenetic gene regulation by Janus kinase 1 in diffuse large B-cell lymphoma", PROCEEDINGS OF THE NATIONAL ACADEMY OF SCIENCES, vol. 113, no. 46, 31 October 2016 (2016-10-31), XP093095234, ISSN: 0027-8424, DOI: 10.1073/pnas.1610970113**
- **LEE YOUNGKYUN ET AL: "The ubiquitin-mediated degradation of Jak1 modulates osteoclastogenesis by limiting interferon-[beta]-induced inhibitory signaling", vol. 111, no. 2, 15 January 2008 (2008-01-15), US, pages 885 - 893, XP093095022, ISSN: 0006-4971, Retrieved from the Internet <URL:https://watermark.silverchair.com/zh800208000885.pdf?token=AQECAHi208BE49Ooan9kkhW_Ercy7Dm3ZL_9Cf3qfKAc485ysgAABGIwggReBgkqhkiG9w0BBwagggRPMIIESwIBADCCBEQGCSqGSIb3DQEHATAeBglghkgBZQMEAS4wEQQM_It5XPyxnSSxg5usAgEQgIIEFep9cZGhVa5B5YqbaHzwY-5bpO9FrBUzbXCzxrqUeY-cnMwXfRvXaDohhvXN2xK0Bb73c3lBm9vqipOULsJ-0> DOI: 10.1182/blood-2007-03-082941**


Remarques:

Le document complet y compris le(s) table(s) de référence et le(s) listage(s) de séquence sont téléchargeables sur le site internet de l’OEB

## Description

*DOMAINE DE L'INVENTION*

La présente invention concerne les séquences nucléotidiques de petits ARN interférents spécifiques du gène JAK1 humain, respectivement. Ces séquences ont été sélectionnées afin de présenter le moins d'effets indésirables possibles tout en maximisant leur potentiel d'inhibition fonctionnelle de l'expression du gène JAK1, pour permettre une utilisation thérapeutique chez l'homme.

*ARRIERE-PLAN TECHNOLOGIQUE*

L'ARN interférence a été décrite pour la première fois en 1998 par Andrew Fire et Craig Mello. Ce processus a lieu lorsqu'un ARN double brin présente une séquence nucléotidique complémentaire d'un ARNm. L'ARN double brin active alors la dégradation de l'ARNm dont il est complémentaire, diminuant ainsi l'expression du gène correspondant. Dans le détail, les ARN double brins présents dans une cellule sont tout d'abord pris en charge par une ribonucléase de type III appelée Dicer, l'« émninceuse ». Celle-ci clive l'ARN double brin toutes les 21 à 25 paires de bases. Dicer transfère alors les petits ARN interférents (pARNi) à un gros complexe multiprotéique, le complexe RISC (RNA-induced silencing complex). Un des brins du pARNi, dit « passager », est éliminé tandis que l'autre (appelé « guide ») dirige le complexe RISC vers les ARNm (ARN messagers, ARN codant transmettant le message du gène dans le cytoplasme) possédant une séquence complémentaire au brin guide. Si la complémentarité entre le pARNi et l'ARNm cible est parfaite, le complexe RISC clive l'ARNm cible qui est alors dégradé et n'est donc plus traduit en protéine. Quelques bases non complémentaires suffisent pour empêcher le clivage.

Les petits ARN interférents, « siARN », sont des duplexes synthétiques de 21 nucléotides qui présentent notamment en position 2 à 8 du brin guide dans le sens 5' - 3' une séquence « graine » particulièrement importante pour assurer leur spécificité. Ces séquences respectent certaines règles de thermodynamique telles que la présence d'une guanine ou d'une cytosine en position 1 du brin passager, une adénine ou un uracile en position 1 du brin guide, d'une composition de la séquence « graine » du brin guide avec un taux minimal de guanine / cytosine, d'un équilibre guanine / cytosine pour les nucléotides 8 à 16 du brin guide.

En 2018, le siARN « Patisiran », commercialisé par la société Alnylam a été approuvé pour une mise sur le marché. Ce siARN est le premier siARN thérapeutique approuvé pour une utilisation chez l'homme. Les concentrations *in vitro* classiquement utilisées sur lignées cellulaires sont de 10 à 20 nM.

La famille JAK (Janus kinase) comprend quatre tyrosine kinases non réceptrices, JAK1, JAK2, JAK3 et Tyk2, qui jouent un rôle essentiel dans la transduction du signal induite par les cytokines et les facteurs de croissance. Les kinases JAK phosphorylées se lient et activent diverses protéines transducteur de signal et activateur de transcription (STAT). Ces protéines STAT se dimérisent et migrent ensuite vers le noyau où elles agissent à la fois comme molécules de signalisation et facteurs de transcription et se lient finalement à des séquences d'ADN spécifiques présentes dans les promoteurs de gènes sensibles aux cytokines. Diverses immunodéficiences et maladies auto-immunes telles que les allergies, l'asthme, l'alopécie areata, le rejet d'allogreffe (allogreffe), l'arthrite rhumatoïde, la sclérose latérale amyotrophique et la sclérose en plaques, ainsi que des cancers solides et hématologiques résultent d'une perturbation de la signalisation dans la voie JAK/STAT.

*RESUME DE L'INVENTION*

L'invention concerne un acide ribonucléique (ARN) double brin (db) comprenant un brin sens et un brin antisens dans lequel :
le brin sens comprend la séquence nucléotidique SEQ ID NO : 7 et le brin antisens comprend la séquence nucléotidique SEQ ID NO : 8, et chaque brin de l'ARNdb comprend au plus 24 nucléotides, pour son utilisation comme médicament.

L'invention concerne en particulier l'ARNdb tel que défini ci-dessus pour son utilisation pour la prévention et/ou le traitement d'une maladie associée à un dysfonctionnement en gain de fonction de la voie de signalisation de la Janus kinase 1 - JAK1).

L'invention concerne en outre une composition pharmaceutique comprenant au moins un ARNdb tel que défini ci-dessus et un véhicule pharmaceutiquement acceptable.

L'invention concerne aussi une méthode *in vitro* pour inhiber l'expression de la Janus kinase 1 (JAK1) dans une cellule, la méthode comprenant :

a. l'introduction dans ladite cellule humaine de l'ARNdb tel que défini ci-dessus ; et
b. le maintien de la cellule produite à l'étape (a) pendant un temps suffisant pour obtenir la dégradation de l'ARNm d'un gène JAK1, inhibant ainsi l'expression de JAK1 dans la cellule.

*DESCRIPTION DETAILLEE*

Les inventeurs ont généré plusieurs séquences de siARN respectant les grands principes de l'interférence à ARN. Les siARN selon la description ont ainsi été conçus de manière à ce que :

i. Le brin guide du siARN présente une homologie de séquence de 50% à 100% avec l'ARNm d'intérêt (JAK1 ou JAK3 humain) ;
ii. Les motifs nucléotidiques connus d'activation des récepteurs Toll-like (TLR) soient éliminés ;
iii. La composition GC / AU en 3' et en 5' soit optimisée afin de favoriser la prise en charge du brin guide dans le complexe RISC par rapport au brin passager ;
iv. Les homologies de séquence potentielles entre la région « graine » du brin guide du siRNA et les séquences ARNm 3'UTR de l'ensemble du génome soient réduites au maximum, afin de minimiser le risque d'obtenir des effets de régulations géniques de type microARN ;
v. De préférence, un phosphate soit ajouté à l'extrémité 5' du brin guide du siARN, afin de favoriser la prise en charge du brin guide par le complexe RISC.

Une stratégie de criblage a ensuite été mise au point consistant à comparer en parallèle différentes séquences siARN, basée sur leur efficacité fonctionnelle d'inhibition de l'expression génique, leur spécificité (pas de modulation directe d'autres gènes), la réduction de leurs effets indésirables (effets off-target, effets immunogènes, effets non spécifiques potentiels). Les inventeurs ont ainsi sélectionné deux séquences de siARN chacune capables de diminuer spécifiquement l'expression du gène et donc de la protéine JAK1 ou JAK3 humaine, sans présenter d'effets indésirables (en particulier pas d'effet sur l'expression de JAK2), dont le point commun est le potentiel thérapeutique, pour traiter une maladie associée à un dysfonctionnement en gain de fonction de la voie de signalisation des Janus kinases, notamment les maladies inflammatoires (dont les maladies inflammatoires chroniques de l'intestin) ou certains cancers.

*Définitions*

Le terme « ARN double brin » ou « ARNdb » désigne un complexe de molécules d'acide ribonucléiques ayant une structure en duplex comprenant deux brins d'acides nucléiques anti-parallèles, essentiellement complémentaires. En général, chaque brin est constitué en majorité ou en totalité de ribonucléotides, mais l'un ou les deux brins peuvent inclure au moins une base non ribonucléotidique, par exemple un désoxyribonucléotide et/ou un nucléotide modifié, par exemple par modification chimique. Les deux brins formant la structure en duplex peuvent être des parties différentes d'une molécule d'ARN plus grande ou peuvent être des molécules d'ARN séparées (siARN). Lorsque les deux brins sont des parties différentes d'une molécule d'ARN plus grande, l'extrémité 3' d'un brin peut être connectée par une chaîne nucléotidique à l'extrémité 5' de l'autre brin pour former une structure en épingle à cheveux (shARN). Alternativement les deux brins peuvent être connectés un moyen de liaison (« linker ») autre qu'une chaîne nucléotidique.

Le « brin antisens » désigne le brin de l'ARNdb qui inclut une région de complémentarité qui est essentiellement complémentaire d'une séquence cible, selon l'invention une séquence cible sur un ARNm de JAK1 ou JAK3.

Le « brin sens » désigne le brin de ARNdb qui inclut une région qui est essentiellement complémentaire d'une région du brin antisens.

Deux séquences dont les bases s'apparient sur toute leur longueur sont totalement complémentaires. Une séquence peut également être essentiellement complémentaire d'une deuxième séquence lorsque les deux séquences s'hybrident et sont totalement complémentaires, ou sont totalement complémentaires sur une partie de leur longueur (par exemple dans le cas d'un complexe double brin avec débordement), ou lorsqu'elles comportent au plus 4, 3, 2, ou 1 mésappariement de base.

Dans l'ensemble de la présente demande, le terme "comprenant" doit être interprété comme englobant toutes les caractéristiques spécifiquement mentionnées, ainsi que des caractéristiques facultatives, additionnelles, non spécifiées. Tel qu'utilisé ici, l'utilisation du terme "comprenant" décrit également le mode de réalisation dans lequel aucune caractéristique autre que les caractéristiques spécifiquement mentionnées n'est présente (c'est-à-dire "consistant en").

*Acide ribonucléique double brin*

Comme il sera décrit plus en détail ci-après, des molécules d'acide ribonucléique (ARN) double brin (db) sont fournies pour inhiber la voie de signalisation JAK/STAT, en particulier chez un être humain ayant une maladie associée à un dysfonctionnement en gain de fonction de la voie de signalisation des Janus kinases (JAK), dans lesquelles l'ARNdb comprend un brin antisens qui est complémentaire à un ARNm résultant de la transcription d'un gène JAK1 ou JAK3.

Selon un mode de réalisation, les deux brins de l'ARNdb sont des molécules d'ARN séparées (siARN). Chaque brin de l'ARNdb comprend 21 nucléotides, et comprend de préférence au plus 24, 23, ou 22 nucléotides. Les deux brins de

l'ARNdb sont de longueurs identiques ou différentes, de préférence de même longueur.

De préférence, chaque brin de l'ARNdb comporte un débordement en 3' de 2 nucléotides ou plus, de préférence de 2 nucléotides.

Selon un mode de réalisation, les deux brins de l'ARNdb sont des parties d'une molécule d'ARN plus grande et constituent par exemple la partie tige d'une structure en épingle à cheveux. Selon ce mode de réalisation, l'ARNdb comprend de préférence au plus 60 nucléotides, par exemple entre 55 et 60 nucléotides au total.

Selon la description l'ARNdb comprend ou consiste en un brin sens et un brin antisens, dans lequel :

le brin sens consiste en la séquence nucléotidique SEQ ID NO : 1 et le brin antisens consiste en la séquence nucléotidique SEQ ID NO : 2 ; ou
le brin sens consiste en la séquence nucléotidique SEQ ID NO : 3 et le brin antisens consiste en la séquence nucléotidique SEQ ID NO : 4 ; ou
le brin sens consiste en la séquence nucléotidique SEQ ID NO : 5 et le brin antisens consiste en la séquence nucléotidique SEQ ID NO : 6 ; ou
le brin sens consiste en la séquence nucléotidique SEQ ID NO : 7 et le brin antisens consiste en la séquence nucléotidique SEQ ID NO : 8 ;
et dans lequel le nucléotide à l'extrémité 5' du brin antisens est phosphorylé.

Selon la description, l'ARNdb est un ARNdb qui réduit l'expression du gène Janus kinase 3 (JAK3) humain et dans lequel le brin sens comprend la séquence nucléotidique SEQ ID NO : 1 et le brin antisens comprend la séquence nucléotidique SEQ ID NO : 2 (HJ3D41), ou le brin sens comprend la séquence nucléotidique SEQ ID NO : 3 et le brin antisens comprend la séquence nucléotidique SEQ ID NO : 4 (HMJ3D1). De préférence, dans ledit ARNdb, le brin sens comprend la séquence nucléotidique SEQ ID NO : 1 et le brin antisens comprend la séquence nucléotidique SEQ ID NO : 2 (HJ3D41). L'ARNdb qui comprend le brin sens comprenant la séquence nucléotidique SEQ ID NO : 3 et le brin antisens comprenant la séquence nucléotidique SEQ ID NO : 4 (HMJ3D1) est en outre spécifique du gène JAK3 murin et est capable de réduire l'expression de ce gène.

Selon la description, l'ARNdb est un ARNdb qui réduit l'expression du gène Janus kinase 1 (JAK1) humain et dans lequel le brin sens comprend la séquence nucléotidique SEQ ID NO : 5 et le brin antisens comprend la séquence nucléotidique SEQ ID NO : 6 (HJ1D2). Selon l'invention, l'ARNdb pour son utilisation comme médicament est un ARNdb qui réduit l'expression du gène Janus kinase 1 (JAK1) humain et dans lequel, le brin sens comprend la séquence nucléotidique SEQ ID NO : 7 et le brin antisens comprend la séquence nucléotidique SEQ ID NO : 8 (HJ1D8) et chaque brin de l'ARNdb comprend au plus 24 nucléotides.

L'ARNdb peut être modifié chimiquement, en particulier pour augmenter sa stabilité ou augmenter la prise en charge du brin guide par le complexe RISC. Des exemples de molécules d'ARNdb modifiée incluent des ARNdb contenant des modifications du squelette ou des liaisons internucléotidiques non naturelles, ou contenant des bases nucléotidiques modifiées.

De manière préférentielle, l'ARNdb est un ARNdb dans lequel le nucléotide à l'extrémité 5' du brin antisens est phosphorylé.

L'ARNdb selon l'invention peut aussi être modifié chimiquement pour former un conjugué, c'est à dire lier de manière covalente un ou plusieurs groupements qui augmentent l'activité, la distribution cellulaire ou l'incorporation cellulaire de l'ARNdb.

L'ARNdb peut être préparé par toute méthode connue de l'homme du métier, par exemple par synthèse chimique ou par génie génétique. Dans ce dernier cas, les ARNdb sont exprimés à partir d'unités transcriptionnelles insérées dans un ou des vecteurs à ADN ou ARN. Ces transgènes peuvent être introduits sous forme de construction linéaire, de plasmide circulaire, ou de vecteur viral puis incorporés dans une cellule hôte où ils sont intégrés au génome cellulaire hôte, ou bien présent sous forme de plasmide extra-chromosomique. N'importe quel vecteur viral capable d'accepter les séquences codant l'ARNdb peut être utilisé, par exemple des vecteurs dérivés d'adénovirus (AV), des virus associés à l'adénovirus (AAV), des rétrovirus (par exemple lentivirus, rhabdovirus), herpes virus, etc. Le tropisme du virus peut être modifié par pseudotypage du vecteur par exemple avec des protéines d'enveloppe ou d'autres antigènes de surface provenant d'autres virus.

Les deux brins du ARNdb peuvent être transcrits sous le contrôle de promoteurs à partir de deux vecteurs d'expression distincts qui sont co-transfectés dans une cellule hôte, ou bien sous le contrôle de promoteurs qui sont chacun sur un même vecteur d'expression. L'ARNdb peut également être exprimé sous forme de deux séquences répétées inversées jointes par une séquence polynucléotidique de liaison pour former une structure en tige et boucle (petit ARN en épingle à cheveux, ou shARN).

Les promoteurs contrôlant l'expression de l'ARNdb présents dans un plasmide ADN ou un vecteur viral peuvent être un promoteur d'ARN polymérase I, II ou III (par exemple U6) eucaryote ou un promoteur procaryote (par exemple le promoteur T7).

Les plasmides ADN pour l'expression d'ARNdb sont typiquement introduits dans les cellules cibles par transfection selon les techniques et à l'aide de véhicules bien connus de l'homme du métier. L'efficacité de transfection peut être contrôlée par exemple en utilisant un marqueur fluorescent tel que la GFP en tant que gène rapporteur.

*Indications médicales*

Un ARNdb selon la description est utilisable comme médicament, en thérapie, en particulier en thérapie humaine, du fait des propriétés i-v listées ci-dessus ayant guidé sa conception et de la sélection qui a été opérée par les inventeurs parmi les 43 siRNA initialement générés pour bloquer l'expression du gène JAK1 ou JAK3 humain et/ou murin. L'ARNdb selon l'invention est utilisable comme médicament pour bloquer l'expression du gène JAK1 pour bloquer l'expression du gène JAK1.

L'ARNdb est plus particulièrement indiqué pour le traitement d'une maladie associée à un dysfonctionnement en gain de fonction de la voie de signalisation des Janus kinases (JAK), en particulier JAK1 et/ou JAK3.

Une méthode de traitement d'une maladie associée à un dysfonctionnement en gain de fonction de la voie de signalisation des Janus kinases (JAK) est ainsi proposée, dans laquelle une quantité thérapeutiquement efficace d'au moins un ARNdb selon la description est administré à un sujet souffrant d'une maladie associée à un dysfonctionnement en gain de fonction de la voie de signalisation JAK, en particulier JAK1 et/ou JAK3. Une méthode de traitement d'une maladie associée à un dysfonctionnement en gain de fonction de la voie de signalisation des Janus kinases (JAK) est ainsi proposée, dans laquelle une quantité thérapeutiquement efficace de l'ARNdb selon l'invention est administré à un sujet souffrant d'une maladie associée à un dysfonctionnement en gain de fonction de la voie de signalisation JAK1.

Le sujet est un mammifère, de préférence un primate, et de manière préférentielle un sujet humain. Le sujet peut également être un rongeur, de préférence une souris.

Les maladies associées à un dysfonctionnement en gain de fonction de la voie de signalisation des Janus kinases (JAK), en particulier de JAK1 et/ou JAK3, incluent en particulier les maladies inflammatoires à médiation immunitaire, dont notamment les maladies inflammatoires chroniques de l'intestin (maladie de Crohn et rectocolite hémorragique), la polyarthrite rhumatoïde, l'alopécie areata, l'uvéite, la dermatite atopique, la spondylarthrite ankylosante, le psoriasis, lupus érythémateux, néphrite lupique, la myélofibrose, le rejet de greffe et la maladie du greffon contre l'hôte ; et les cancers, notamment les leucémies (en particulier leucémie aiguë lymphoblastique (LAL), et leucémie aiguë myéloblastique (LAM)) et les cancers à tumeurs solides. De manière préférentielle, un ARNdb selon l'invention est indiqué pour le traitement des maladies inflammatoires à médiation immunitaire, dont notamment les maladies inflammatoires chroniques de l'intestin, de la myélofibrose, du rejet de greffe, et des cancers.

Pour son utilisation thérapeutique, l'ARNdb est généralement formulé dans une composition pharmaceutique qui comprend au moins un ARNdb selon l'invention et un véhicule pharmaceutiquement acceptable.

Selon un mode de réalisation, la composition pharmaceutique comprend au moins un ARNdb inhibiteur de JAK 1 selon l'invention.

Selon un autre aspect, la composition pharmaceutique comprend au moins un ARNdb inhibiteur de JAK 3 selon la description.

Selon encore un autre aspect, la composition pharmaceutique comprend au moins un ARNdb inhibiteur de JAK 1 et au moins un ARNdb inhibiteur de JAK 3 selon l'invention, par exemple :

- un ARNdb dans lequel le brin sens comprend la séquence nucléotidique SEQ ID NO : 1 et le brin antisens comprend la séquence nucléotidique SEQ ID NO : 2 (HJ3D41), et (i) un ARNdb dans lequel le brin sens comprend la séquence nucléotidique SEQ ID NO : 7 et le brin antisens comprend la séquence nucléotidique SEQ ID NO : 8 (HJ1D8) ou (ii) un ARNdb dans lequel le brin sens comprend la séquence nucléotidique SEQ ID NO : 5 et le brin antisens comprend la séquence nucléotidique SEQ ID NO : 6 (HJ1D2) ; ou
- un ARNdb dans lequel le brin sens comprend la séquence nucléotidique SEQ ID NO : 3 et le brin antisens comprend la séquence nucléotidique SEQ ID NO : 4 (HMJ3D1), et (i) un ARNdb dans lequel le brin sens comprend la séquence nucléotidique SEQ ID NO : 7 et le brin antisens comprend la séquence nucléotidique SEQ ID NO : 8 (HJ1D8) ou (ii) un ARNdb dans lequel le brin sens comprend la séquence nucléotidique SEQ ID NO : 5 et le brin antisens comprend la séquence nucléotidique SEQ ID NO : 6 (HJ1D2).

La composition pharmaceutique selon l'invention peut également comprendre au moins un ARNdb selon l'invention, au moins un autre principe actif, par exemple un agent anticancéreux, et un véhicule pharmaceutiquement acceptable.

Selon encore un autre mode de réalisation, l'invention concerne un ARNdb inhibiteur de JAK 1 selon l'invention pour son utilisation comme médicament, ou pour son utilisation pour la prévention et/ou le traitement d'une maladie associée à un dysfonctionnement en gain de fonction de la voie de signalisation des Janus kinases (JAK), en combinaison avec un ARNdb inhibiteur de JAK 3 selon la description ou un autre principe actif, par exemple un agent anticancéreux.

Selon ce mode de réalisation, l'ARNdb inhibiteur de JAK 1, et l'ARNdb inhibiteur de JAK 3 selon la description ou l'autre principe actif, peuvent être formulés dans une même composition pharmaceutique ou bien de manière séparée, dans des compositions pharmaceutiques distinctes. Ils peuvent être administrés simultanément ou séparément, par exemple de manière espacée dans le temps. Selon ce mode de réalisation, l'ARNdb inhibiteur de JAK 1 est administré à un sujet qui traité un ARNdb inhibiteur de JAK 3 selon la description ou un autre principe actif.

Selon encore un autre mode de réalisation, l'invention concerne un ARNdb inhibiteur de JAK 3 selon la description pour son utilisation comme médicament, ou pour son utilisation pour la prévention et/ou le traitement d'une maladie associée à un dysfonctionnement en gain de fonction de la voie de signalisation des Janus kinases (JAK), en combinaison avec un ARNdb inhibiteur de JAK 1 selon l'invention ou un autre principe actif, par exemple un agent anticancéreux. Selon ce mode de réalisation, l'ARNdb inhibiteur de JAK 3, et l'ARNdb inhibiteur de JAK 1 selon l'invention ou l'autre principe actif, peuvent être formulés dans une même composition pharmaceutique ou bien de manière séparée, dans des compositions pharmaceutiques distinctes. Ils peuvent être administrés simultanément ou séparément, par exemple de manière espacée dans le temps. Selon ce mode de réalisation, l'ARNdb inhibiteur de JAK 3 est administré à un sujet qui traité un ARNdb inhibiteur de JAK 1 selon l'invention ou un autre principe actif.

La composition pharmaceutique est formulée en fonction de sa voie d'administration qui peut être par exemple une administration locale ou une administration systémique, telle que la voie parentérale, ou la voie intraveineuse (iv).

La composition pharmaceutique est administrée avec un dosage suffisant en ARNdb pour induire une inhibition de l'expression de JAK1 et/ou JAK3.

La détermination de la voie d'administration et du dosage adapté en fonction du sujet est à la portée de l'homme du métier.

*Méthode pour inhiber JAK1 ou JAK3 dans une cellule*

La demande décrit également une méthode, *in vivo* ou *in vitro,* pour inhiber l'expression de la Janus kinase 1 (JAK1) ou la Janus kinase 3 (JAK3) dans une cellule, la méthode comprenant :

a. l'introduction dans ladite cellule de l'ARNdb selon l'invention ; et
b. le maintien de la cellule produite à l'étape (a) pendant un temps suffisant pour obtenir la dégradation de l'ARNm d'un gène JAK1 ou JAK3, inhibant ainsi l'expression de JAK1 ou JAK3 dans la cellule.

La cellule est de préférence une cellule humaine ou murine.

Selon un mode de réalisation, à l'étape a, l'ARNdb est mis en contact avec ladite cellule à une concentration supérieure ou égale à 5 pM, de préférence de 5 pM à 10 nM, ou encore de 5 pM à 1 nM, ou encore de 5 pM à 100 pM, ou encore de 5 pM à 50 pM de préférence encore de 5 pM à 25 pM.

Un réactif de transfection (par exemple des lipides cationiques, tels que la Lipofectamine) est typiquement utilisé pour faciliter la transfection de la cellule par l'ARNdb.

Lorsque la méthode est mise en œuvre *in vitro,* à l'étape b) la cellule est maintenue dans un milieu de culture approprié à sa survie et/ou propagation. Le choix d'un tel milieu est à la portée de l'homme du métier.

Les modes de réalisation suivants font partie de l'invention :

**Item 1.** Acide ribonucléique (ARN) double brin (db) comprenant un brin sens et un brin antisens, dans lequel :

le brin sens comprend la séquence nucléotidique SEQ ID NO : 1 et le brin antisens comprend la séquence nucléotidique SEQ ID NO : 2 ; ou
le brin sens comprend la séquence nucléotidique SEQ ID NO : 3 et le brin antisens comprend la séquence nucléotidique SEQ ID NO : 4 ; ou
le brin sens comprend la séquence nucléotidique SEQ ID NO : 5 et le brin antisens comprend la séquence nucléotidique SEQ ID NO : 6 ; ou
le brin sens comprend la séquence nucléotidique SEQ ID NO : 7 et le brin antisens comprend la séquence nucléotidique SEQ ID NO : 8 ;
pour son utilisation comme médicament.

**Item 2.** Acide ribonucléique (ARN) double brin (db) comprenant un brin sens et un brin antisens, dans lequel :

le brin sens comprend la séquence nucléotidique SEQ ID NO : 1 et le brin antisens comprend la séquence nucléotidique SEQ ID NO : 2 ; ou
le brin sens comprend la séquence nucléotidique SEQ ID NO : 3 et le brin antisens comprend la séquence nucléotidique SEQ ID NO : 4 ; ou

le brin sens comprend la séquence nucléotidique SEQ ID NO : 5 et le brin antisens comprend la séquence nucléotidique SEQ ID NO : 6 ; ou
le brin sens comprend la séquence nucléotidique SEQ ID NO : 7 et le brin antisens comprend la séquence nucléotidique SEQ ID NO : 8 ;
pour son utilisation pour la prévention et/ou le traitement d'une maladie associée à un dysfonctionnement en gain de fonction de la voie de signalisation de la Janus kinase 1 (JAK1) ou de la Janus kinase 3 (JAK3).

**Item 3.** ARNdb pour l'utilisation selon l'item 2, où la maladie est sélectionnée dans le groupe constitué des maladies inflammatoires à médiation immunitaire et des cancers.
**Item 4.** ARNdb pour l'utilisation selon l'un quelconque des items 1 à 3, où ledit ARNdb réduit l'expression de la Janus kinase 3 (JAK3) et dans lequel :

le brin sens comprend la séquence nucléotidique SEQ ID NO : 1 et le brin antisens comprend la séquence nucléotidique SEQ ID NO : 2 ; ou
le brin sens comprend la séquence nucléotidique SEQ ID NO : 3 et le brin antisens comprend la séquence nucléotidique SEQ ID NO : 4.

**Item 5.** ARNdb pour l'utilisation selon l'un quelconque des items 1 à 3, où ledit ARNdb réduit l'expression de la Janus kinase 1 (JAK1) et dans lequel :

le brin sens comprend la séquence nucléotidique SEQ ID NO : 5 et le brin antisens comprend la séquence nucléotidique SEQ ID NO : 6 ; ou
le brin sens comprend la séquence nucléotidique SEQ ID NO : 7 et le brin antisens comprend la séquence nucléotidique SEQ ID NO : 8.

**Item 6.** ARNdb pour l'utilisation selon l'un quelconque des items 1 à 5, pour une utilisation chez un sujet humain.
**Item 7.** Acide ribonucléique (ARN) double brin (db) comprenant un brin sens et un brin antisens, utilisable en thérapie humaine, dans lequel :

le brin sens comprend la séquence nucléotidique SEQ ID NO : 1 et le brin antisens comprend la séquence nucléotidique SEQ ID NO : 2 ; ou
le brin sens comprend la séquence nucléotidique SEQ ID NO : 3 et le brin antisens comprend la séquence nucléotidique SEQ ID NO : 4 ; ou
le brin sens comprend la séquence nucléotidique SEQ ID NO : 5 et le brin antisens comprend la séquence nucléotidique SEQ ID NO : 6 ; ou
le brin sens comprend la séquence nucléotidique SEQ ID NO : 7 et le brin antisens comprend la séquence nucléotidique SEQ ID NO : 8.

**Item 8.** Composition pharmaceutique comprenant au moins un ribonucléique (ARN) double brin (db) comprenant un brin sens et un brin antisens dans lequel :

le brin sens comprend la séquence nucléotidique SEQ ID NO : 1 et le brin antisens comprend la séquence nucléotidique SEQ ID NO : 2 ; ou
le brin sens comprend la séquence nucléotidique SEQ ID NO : 3 et le brin antisens comprend la séquence nucléotidique SEQ ID NO : 4 ; ou
le brin sens comprend la séquence nucléotidique SEQ ID NO : 5 et le brin antisens comprend la séquence nucléotidique SEQ ID NO : 6 ; ou
le brin sens comprend la séquence nucléotidique SEQ ID NO : 7 et le brin antisens comprend la séquence nucléotidique SEQ ID NO : 8 ;
et un véhicule pharmaceutiquement acceptable.

**Item 9.** Méthode *in vitro* pour inhiber l'expression de la Janus kinase 1 (JAK1) ou la Janus kinase 3 (JAK3) dans une cellule, la méthode comprenant :

a. l'introduction dans ladite cellule d'un acide ribonucléique (ARN) double brin (db), ledit ARNdb comprenant un brin sens et un brin antisens dans lequel :

le brin sens comprend la séquence nucléotidique SEQ ID NO : 1 et le brin antisens comprend la séquence

nucléotidique SEQ ID NO : 2 ; ou
le brin sens comprend la séquence nucléotidique SEQ ID NO : 3 et le brin antisens comprend la séquence nucléotidique SEQ ID NO : 4 ; ou
le brin sens comprend la séquence nucléotidique SEQ ID NO : 5 et le brin antisens comprend la séquence nucléotidique SEQ ID NO : 6 ; ou
le brin sens comprend la séquence nucléotidique SEQ ID NO : 7 et le brin antisens comprend la séquence nucléotidique SEQ ID NO : 8 ; et

b. le maintien de la cellule produite à l'étape (a) pendant un temps suffisant pour obtenir la dégradation de l'ARNm d'un gène JAK1 ou JAK3, inhibant ainsi l'expression de JAK1 ou JAK3 dans la cellule.

**Item 10.** Méthode *in vitro* selon l'item 9, dans laquelle, à l'étape a, ledit ARNdb est mis en contact avec ladite cellule à une concentration de 5 pM à 10 nM.
**Item 11.** ARNdb pour l'utilisation selon l'un quelconque des items 1 à 6, ARNdb selon la revendication 7, composition pharmaceutique selon la revendication 8, ou méthode selon la revendication 9 ou 10, dans lequel ou laquelle le nucléotide à l'extrémité 5' du brin antisens de l'ARNdb est phosphorylé.
**Item 12.**ARNdb pour l'utilisation selon l'un quelconque des items 1 à 6 et 11, ARNdb selon l'item 7 ou 11, composition pharmaceutique selon l'item 8 ou 11, ou méthode selon l'un quelconque des items 9 à 11, dans lequel ou laquelle chaque brin de l'ARNdb comprend au plus 24, 23 ou 22 nucléotides.
**Item 13.**ARNdb pour l'utilisation selon l'un quelconque des items 1 à 6 et 11-12, ARNdb selon l'item 7, 11 ou 12, composition pharmaceutique selon l'un quelconque des items 8 et 11 à 12, ou méthode selon l'un quelconque des items 9 à 12, dans lequel ou laquelle les deux brins de l'ARNdb sont de longueur identique.
**Item 14.** ARNdb pour l'utilisation selon l'un quelconque des items 1 à 6 et 11 à 14, ARNdb selon l'un quelconque des items 7 et 11 à 13, composition pharmaceutique selon l'un quelconque des items 8 et 11 à 13, ou méthode selon l'un quelconque des items 9 à 13, dans lequel ou laquelle l'ARNdb comprend au plus 60 nucléotides.

La présente invention sera illustrée plus en détail par les figures et exemples ci-dessous.

## FIGURES

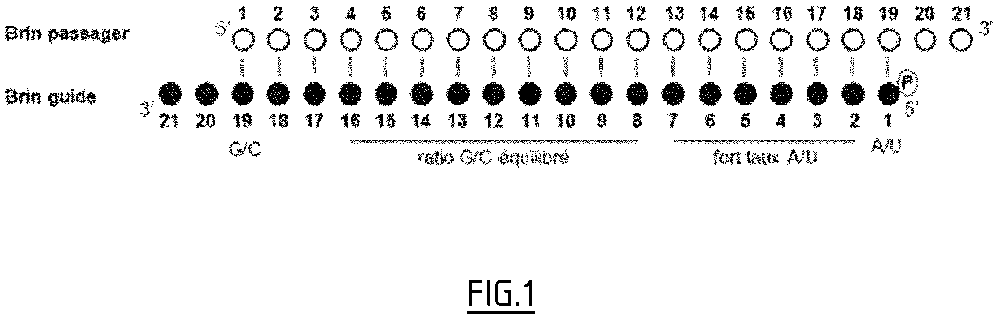

*La Figure 1 représente la structure typique d'un siARN selon l'invention, comportant un débordement en 3', de 2 nucléotides non appariés, et de préférence un groupement phosphate à l'extrémité 5' du brin guide. Le brin passage correspond au brin sens, et le brin guide au bris antisens.*
*La Figure 2 représente les régions d'hybridation, sur la partie codante du gène JAK1, des différents siARN inhibiteurs de JAK1 testés.*
*La Figure 3 représente les régions d'hybridation, sur la partie codante du gène JAK3, des différents siARN inhibiteurs de JAK3 testés*
*La Figure 4 représente les résultats de 3 expériences indépendantes de mesure du niveau d'expression génique JAK1, JAK2, JAK3 ou TYK2 dans des cellules* Caco-2 *transfectées avec 10 nM de siARN (ciblant JAK1).*
*La Figure 5 représente les résultats de 3 expériences indépendantes de mesure du niveau d'expression génique JAK1, JAK2, JAK3 ou TYK2 dans des cellules* Caco-2 *transfectées avec 10 nM de siARN (ciblant JAK3).*
*La Figure 6 représente les résultats de quantification de l'expression de JAK1 dans des cellules PC3 transfectées avec 12,5 pM de siARN.*
*La Figure 7 représente les résultats de quantification de l'expression de JAK3 dans des cellules PC3 surexprimant de façon stable JAK3 transfectées avec 1 nM de siARN.*
*La Figure 8 représente l'analyse de l'expression de JAK1, JAK3, ou de GAPDH, par Western blot dans des cellules PC3 surexprimant de façon stable JAK3 transfectées avec 0,5 pM à 12,5 pM de siARN.*
*La Figure 9 représente l'expression de gènes cibles potentiels dans des cellules T47D transfectées avec 10nM de siARN HJ1D2 ciblant JAK1 ou d'un siARN contrôle (siAS).*
*La Figure 10 représente l'expression de gènes cibles potentiels dans des cellules T47D transfectées avec 10nM de siARN HJ1D8 ciblant JAK1 ou d'un siARN contrôle (siAS).*
*La Figure 11 représente l'expression de gènes cibles potentiels dans des cellules T47D ou* PC3 *transfectées avec 10nM de siARN HJ3D41 ciblant JAK3 ou d'un siARN contrôle (siAS).*
*La Figure 12 représente l'expression de gènes cibles potentiels dans des cellules T47D transfectées avec 10nM de siARN HMJ3D1 ciblant JAK3 ou d'un siARN contrôle (siAS).*
*La Figure 13 montre l'effet des siARN selon l'invention ou contrôle sur la prolifération cellulaire (a), le métabolisme de*

*l'ATP (b), et l'apoptose (c), et (d). « opti » désigne le milieu OPTI-MEM utilisé pour réaliser le mixte lors de la transfection, et « lipo » désigne l'agent de transfection utilisé (LipofectamineRNAimax).*

## EXEMPLES

**Exemple 1** : **Identité des siARN.**

Plusieurs séquences siARN conçues pour inhiber l'expression du gène JAK1 ou J1K3 humain, ou humain et murin, ont été comparées entre elles ainsi qu'avec plusieurs séquences siARN commercialisées connues pour inhiber l'expression des gènes JAK1 ou JAK3 humains. La nomenclature des siARN est la suivante : H pour *Homo Sapiens* (espèce concernée), M pour *Mus musculus,* J1 pour JAK1 : transcrit ciblé (et J3 pour JAK3), puis un numéro unique Dx pour caractériser la séquence. Ainsi siHJ1D8 est la séquence siARN qui cible le gène humain JAK1, dont le numéro unique est D8. siHMJ3D1 est la séquence siARN qui cible les gènes humain et murin JAK3, dont le numéro unique est D8. « A », « Q », et « H » désignent les siARN commercialisés respectivement par la société Ambion, Qiagen, ou Dharmacon.

Plusieurs approches expérimentales ont été combinées afin de proposer deux séquences de siARN les plus efficaces et spécifiques possibles tout en présentant le moins d'effets indésirables possibles afin de permettre leur utilisation thérapeutique (voir Tableaux 1-2).

Tableau 1. Séquences siARN ciblant JAK3 humain

| Nom | Séquence sens (passager) | Séquence anti-sens (guide) |
|---|---|---|
| HJ3D41 | CGACUUUCCAGAAAUCGUAGA (SEQ ID NO: 1) | UACGAUUUCUGGAAAGUCGCA (SEQ ID NO: 2) |
| HMJ3D1 | GCGUGGAGCUGUGCCGCUAUG (SEQ ID NO:3) | UAGCGGCACAGCUCCACGCUG (SEQ ID NO: 4) |

Tableau 2. Séquences siARN ciblant JAK1 humain

| Nom | Séquence sens (passager) | Séquence anti-sens (guide) |
|---|---|---|
| HJ1D2 | GGAUUACAAGGAUGACGAAGG (SEQ ID NO: 5) | UUCGUCAUCCUUGUAAUCCAU (SEQ ID NO: 6) |
| HJ1D8 | GGACAUCAGCUACAAGCGAUA (SEQ ID NO: 7) | UCGCUUGUAGCUGAUGUCCUU (SEQ ID NO: 8) |

Les inventeurs ont comparé des dizaines de séquences, originales ou déjà commercialisées, afin de faire émerger deux siARN pouvant cibler JAK1 ou JAK3 pour une utilisation thérapeutique.

Il est intéressant de remarquer que parmi les siARN générés selon les principes de l'interférence à ARN, la séquence siARN siHJ1D64 n'était en fait pas efficace pour inhiber l'expression de JAK1.

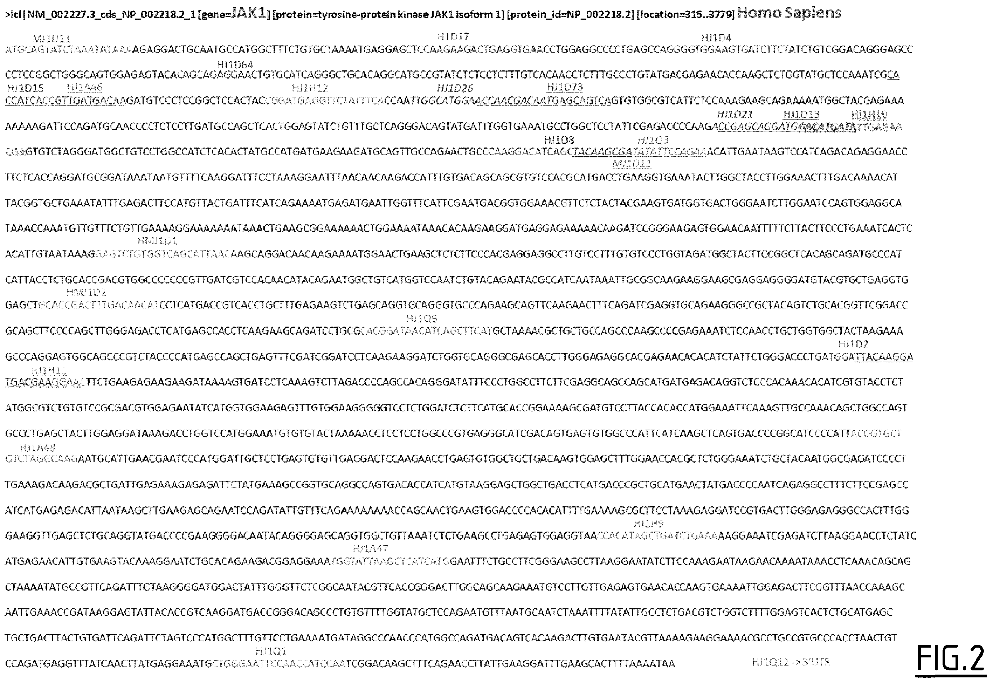

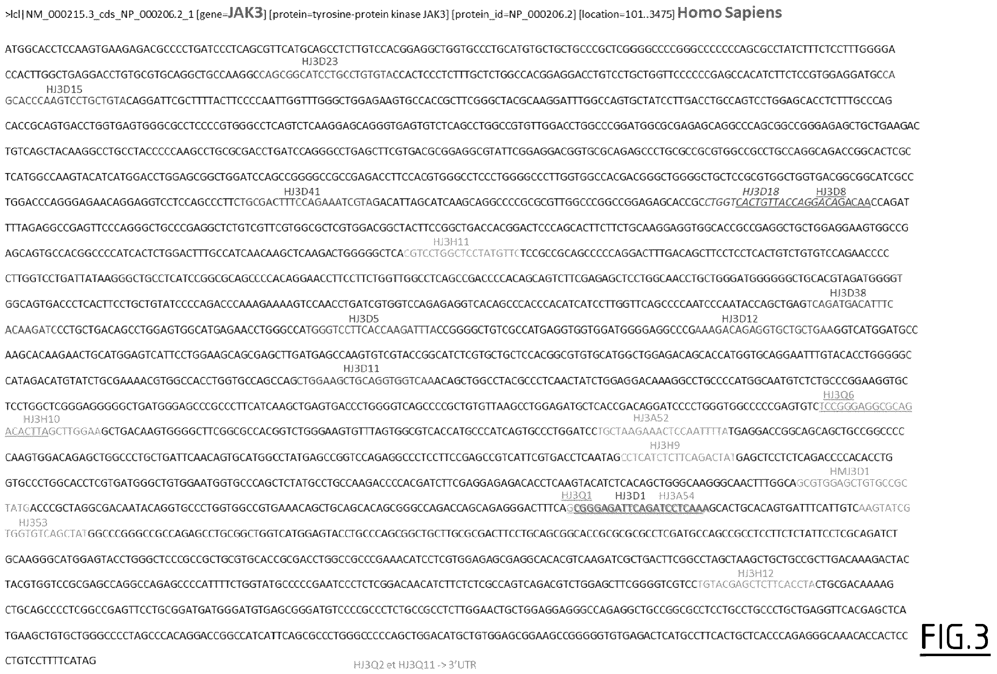

Le positionnement des siARN testés sur les gènes JAK1 et JAK3 est montré en Figures 2 et 3.

Tableau 3 : Synthèse des expériences réalisées pour tester les siARN dirigés contre JAK1 humain.

| | Western Blot | | | | | | | RT-QPCR | | | | |
|---|---|---|---|---|---|---|---|---|---|---|---|---|
| | Caco2 avec siARN 10nM Exp1 | Caco2 avec siARN 10nM Exp2 | Caco2 avec siARN 1nM | Caco2 avec siARN 0,2nM Exp1 | Caco2 avec siARN 0,2nM Exp2 | Caco2 avec siARN 0,2nM Exp3 | **inhibition moyenne Jak1 à 0,2nM Exp1-2-3** | Caco2 avec siARN 0,2nM Exp1 | Caco2 avec siARN 0,2nM Exp2 | Caco2 avec siARN 0,2nM Exp3 | **inhibition moyenne ARNm Jak1 à 0,2nM n=3** | **Score d'efficacité pondéré moyen WB 0,2nM@3, QPCR 0,2nM@2** |
| **siARN** | % inhib. Jak1 | % inhib. Jak1 | % inhib. Jak1 | % inhib. Jak1 | % inhib. Jak1 | % inhib. Jak1 | inhibition moyenne Jak1 à 0,2nM | % inhib. ARNm Jak1 | % inhib. ARNm Jak1 | % inhib. ARNm Jak1 | % inhib. Moyenne ARNm Jak1 | Score d'efficacité pondéré |
| **AS** | 0,0 | 0,0 | 0,0 | 0,0 | 0,0 | 0,0 | **0,0** | 0,0 | 0,0 | 0,0 | **0,0** | **0,0** |
| **HJ1A46** | 96,7 | NA | 92,3 | 73,8 | NA | NA | **NA** | 58,8 | NA | NA | **NA** | **NA** |
| **HJ1A47** | 97,5 | NA | 93,2 | 82,0 | NA | NA | **NA** | NA | NA | NA | **NA** | **NA** |
| **HJ1A48** | NA | 97,0 | 91,3 | 33,5 | NA | NA | **NA** | NA | NA | NA | **NA** | **NA** |
| **HJ1Q1** | 94,0 | 89,4 | 91,6 | 72,1 | NA | NA | **NA** | NA | NA | NA | **NA** | **NA** |
| **HJ1Q3** | 95,1 | 96,1 | 90,2 | 69,1 | NA | NA | **NA** | NA | NA | NA | **NA** | **NA** |
| **HJ1Q6** | 94,9 | 93,7 | 93,7 | 78,2 | NA | NA | **NA** | 70,6 | NA | NA | **70,6** | **NA** |
| **HJ1Q12** | 95,0 | 88,8 | 83,4 | 72,3 | NA | NA | **NA** | 0,0 | NA | NA | **0,0** | **NA** |
| **HJ1H9** | 96,7 | 98,9 | 85,0 | 78,1 | 82,1 | 88,2 | **82,8** | 70,1 | 45,9 | 89,0 | **68,3** | **77,0** |
| **HJ1H10** | 80,2 | 85,6 | NA | NA | NA | NA | **NA** | NA | NA | NA | **NA** | **NA** |
| **HJ1H11** | 92,3 | 92,9 | 81,8 | 62,7 | NA | NA | **62,7** | NA | NA | NA | **NA** | **NA** |
| **HJ1H12** | 94,6 | 90,3 | 83,6 | 75,1 | NA | NA | **75,1** | NA | NA | NA | **NA** | **NA** |
| **HJ1D2** | NA | NA | 91,2 | 76,5 | 94,2 | 92,7 | **87,8** | 77,8 | 91,4 | 90,6 | **86,6** | **87,3** |
| **HJ1D4** | NA | NA | 93,1 | 85,5 | 97,0 | 91,9 | **91,5** | 73,1 | 80,7 | 88,3 | **80,7** | **87,1** |
| **HJ1D8** | NA | NA | 79,3 | 88,3 | 93,5 | 94,9 | **92,2** | 71,0 | 85,2 | 93,3 | **83,2** | **88,6** |
| **HJ1D13** | NA | NA | 88,0 | 76,6 | 61,7 | 95,9 | **78,1** | 43,5 | 55,8 | 83,9 | **61,0** | **71,3** |
| **HJ1D15** | NA | NA | 88,8 | 80,3 | 79,3 | 98,0 | **85,9** | 43,0 | 79,9 | 86,7 | **69,9** | **79,5** |
| **HJ1D17** | NA | NA | 87,7 | 74,3 | 96,7 | 99,9 | **90,3** | 48,4 | 84,0 | 83,5 | **71,9** | **83,0** |

(suite)

| | Western Blot | | | | | | | | RT-QPCR | | | | | |
|---|---|---|---|---|---|---|---|---|---|---|---|---|---|---|
| | **Caco2 avec siARN 10nM Exp1** | **Caco2 avec siARN 10nM Exp2** | **Caco2 avec siARN 1nM** | **Caco2 avec siARN 0,2nM Exp1** | **Caco2 avec siARN 0,2nM Exp2** | **Caco2 avec siARN 0,2nM Exp3** | **inhibition moyenne Jak1 à 0,2nM Exp1-2-3** | | **Caco2 avec siARN 0,2nM Exp1** | **Caco2 avec siARN 0,2nM Exp2** | **Caco2 avec siARN 0,2nM Exp3** | **inhibition moyenne ARNm Jak1 à 0,2nM n=3** | | **Score d'efficacité pondéré moyen WB 0,2nM@3, QPCR 0,2nM@2** |
| **siARN** | **% inhib. Jak1** | **% inhib. Jak1** | **% inhib. Jak1** | **% inhib. Jak1** | **% inhib. Jak1** | % **inhib. Jak1** | **inhibition moyenne Jak1 à 0,2nM** | | **% inhib. ARNm Jak1** | **% inhib. ARNm Jak1** | **% inhib. ARNm Jak1** | **% inhib. Moyenne ARNm Jak1** | | **Score d'efficacité pondéré** |
| **HJ1D21** | NA | NA | 84,0 | 81,2 | 92,7 | 99,5 | **91,1** | | 50,2 | 83,3 | 90,3 | **74,6** | | **84,5** |
| **HJ1D26** | NA | NA | 76,5 | 65,3 | 40,7 | 97,5 | **67,8** | | 37,9 | 74,5 | 63,1 | **58,5** | | **64,1** |
| **HJ1D64** | NA | NA | -7,9 | NA | NA | NA | **NA** | | -97,4 | NA | NA | **NA** | | **NA** |
| **HJ1D73** | NA | NA | NA | 60,8 | 87,7 | 98,9 | **82,5** | | 36,2 | 90,0 | 77,9 | **68,0** | | **76,7** |
| **HMJ1D1** | NA | NA | NA | 83,2 | 95,4 | 95,6 | **91,4** | | 74,1 | 81,2 | 76,8 | **77,4** | | **85,8** |
| **HMJ1D2** | NA | NA | NA | NA | NA | 82,0 | **82,0** | | NA | NA | NA | **NA** | | **NA** |
| **MJ1D11** | NA | NA | NA | NA | 86,9 | 82,0 | **84,4** | | NA | 79,2 | 79,2 | **79,2** | | **82,3** |

| | Quantification de la fluorescence (microscope CellInsigh) | | | Western Blot | | | | | | FACS | | | | RT-QPCR | | | | |
|---|---|---|---|---|---|---|---|---|---|---|---|---|---|---|---|---|---|---|
| | PC3* avec siARN 10nM | PC3* avec siARN 1nM | Zscore moyen pondéré Exp à 10nM=1 et Exp à 1nM=2 | PC3* avec siARN 1nM | PC3* avec siARN 0,2nM | PC3* avec siARN 0,2nM Exp1 | PC3* avec siARN 0,2nM Exp2 | PC3* avec siARN 0,2nM Exp3 | Inhibition moyenne JAK3 à 0,2nM Exp1-2-3 | PC3* avec siARN 0,2nM Exp1 | PC3* avec siARN 0,2nM Exp2 | PC3* avec siARN 0,2nM Exp3 | Inhibition moyenne à 0,2nM Exp1-2-3 | PC3* avec siARN 0,2nM Exp1 | PC3* avec siARN 0,2nM Exp2 | PC3* avec siARN 0,2nM Exp3 | Inhibition moyenne JAK3 à 0,2nM Exp1-2-3 | score d'efficacité pondéré avec HCS@1 FACS@1, WB_E3-1 0,2nM@2, WB_E8 0,2nM@3, QPCR 0.2@3 |
| siARN | Zscore | Zscore | Zscore moyen pondéré | % inhib. Jak3 | % inhib. Jak3 | % inhib. Jak3 | % inhib. Jak3 | % inhib. Jak3 | Moy. Inhib. Jak3 à 0,2nM | % inhib. Fluo. | % inhib. Fluo. | % inhib. Fluo. | Moy. Inhib. Jak3 à 0,2nM | % inhib. ARNm Jak3 | % inhib. ARNm Jak3 | % inhib. ARNm Jak3 | Moy. Inhib. Jak3 à 0,2nM | score d'efficacité pondéré |
| AS | 0,0 | 0,0 | **0,0** | 0,0 | 0,0 | 0,0 | 0,0 | 0,0 | **0,0** | 0,0 | 0,0 | 0,0 | **0,0** | 0,0 | 0,0 | 0,0 | **0,0** | **0,0** |
| HJ3A52 | -0,5 | -1,2 | **-1,0** | 61,0 | 72,0 | NA | NA | NA | **NA** | NA | NA | NA | **NA** | NA | NA | NA | **NA** | **NA** |
| HJ3A53 | -1,0 | -1,2 | **-1,1** | 78,9 | 85,8 | 85,4 | 74,3 | 85,8 | **81,8** | 54,1 | 51,3 | 55,0 | **53,5** | 94,1 | 89,6 | 93,1 | **92,3** | **74,8** |
| HJ3A54 | -0,9 | -1,3 | **-1,2** | 58,5 | 74,2 | NA | NA | | **NA** | NA | NA | NA | **NA** | NA | NA | NA | **NA** | **NA** |
| HJ3Q1 | -1,0 | -2,2 | **-1,8** | 78,7 | 83,3 | 83,0 | 82,3 | 77,7 | **81,0** | 54,1 | 57,1 | 64,4 | **58,5** | 89,2 | 60,3 | 93,6 | **81,0** | **71,3** |
| HJ3Q2 | 1,2 | 1,0 | **1,0** | -30,2 | -0,4 | NA | NA | NA | **NA** | NA | NA | NA | **NA** | NA | NA | NA | **NA** | **NA** |
| HJ3Q6 | -0,4 | -0,8 | **-0,7** | 59,8 | 52,1 | 38,3 | 3,0 | NA | **20,6** | NA | NA | NA | **NA** | NA | NA | NA | **NA** | **NA** |
| HJ3Q11 | 1,0 | 1,2 | **1,1** | 20,1 | -4,3 | NA | NA | NA | **NA** | NA | NA | NA | **NA** | NA | NA | NA | **NA** | **NA** |
| HJ3H9 | -0,5 | -1,2 | **-1,0** | 83,0 | 72,4 | 81,6 | 83,3 | 89,7 | **84,9** | NA | NA | NA | **NA** | NA | NA | NA | **NA** | **NA** |
| HJ3H10 | -0,1 | -1,1 | **-0,8** | 80,7 | 68,2 | 40,8 | 76,7 | 74,8 | **64,1** | NA | NA | NA | **NA** | NA | NA | NA | **NA** | **NA** |
| HJ3H11 | -0,3 | -0,7 | **-0,5** | 74,0 | 73,3 | NA | NA | NA | **NA** | NA | NA | NA | **NA** | NA | NA | NA | **NA** | **NA** |
| HJ3H12 | -0,4 | -1,1 | **-0,8** | 83,3 | 82,6 | 79,8 | 23,2 | 93,3 | **65,4** | 51,4 | 52,4 | 56,4 | **53,4** | 86,7 | 95,3 | 95,2 | **92,4** | **69,3** |
| HJ3D1 | -0,5 | -1,8 | **-1,3** | 67,9 | 70,6 | 77,7 | 75,1 | 85,0 | **79,3** | 49,2 | 50,8 | 54,0 | **51,3** | 96,8 | 81,7 | 93,5 | **90,7** | **70,4** |

| | Quantification de la fluorescence (microscope CellInsigh) | | | Western Blot | | | | | | FACS | | | | RT-QPCR | | | | |
|---|---|---|---|---|---|---|---|---|---|---|---|---|---|---|---|---|---|---|
| | PC3* avec siARN 10nM | PC3* avec siARN 1nM | Zscore moyen pondéré Exp à 10nM=1 et Exp à 1nM=2 | PC3* avec siARN 1nM | PC3* avec siARN 0,2nM | PC3* avec siARN 0,2nM Exp1 | PC3* avec siARN 0,2nM Exp2 | PC3* avec siARN 0,2nM Exp3 | Inhibition moyenne JAK3 à 0,2nM Exp1-2-3 | PC3* avec siARN 0,2nM Exp1 | PC3* avec siARN 0,2nM Exp2 | PC3* avec siARN 0,2nM Exp3 | Inhibition moyenne à 0,2nM Exp1-2-3 | PC3* avec siARN 0,2nM Exp1 | PC3* avec siARN 0,2nM Exp2 | PC3* avec siARN 0,2nM Exp3 | Inhibition moyenne JAK3 à 0,2nM Exp1-2-3 | score d'efficacité pondéré avec HCS@1 FACS@1, WB_E3-1 0,2nM@2, WB_E8 0,2nM@3, QPCR 0.2@3 |
| siARN | Zscore | Zscore | Zscore moyen pondéré | % inhib. Jak3 | % inhib. Jak3 | % inhib. Jak3 | % inhib. Jak3 | % inhib. Jak3 | Moy. Inhib. Jak3 à 0,2nM | % inhib. Fluo. | % inhib. Fluo. | % inhib. Fluo. | Moy. Inhib. Jak3 à 0,2nM | % inhib. ARNm Jak3 | % inhib. ARNm Jak3 | % inhib. ARNm Jak3 | Moy. Inhib. Jak3 à 0,2nM | score d'efficacité pondéré |
| HJ3D5 | -0,5 | -3,3 | **-2,4** | 53,9 | 64,1 | 81,3 | 78,3 | 86,7 | **82,1** | 42,1 | 45,0 | 51,5 | **46,2** | 92,4 | 67,2 | 95,2 | **84,9** | **67,8** |
| HJ3D8 | -0,3 | 0,6 | **0,3** | 23,2 | 57,8 | 65,0 | 58,1 | 71,1 | **64,7** | NA | NA | NA | **NA** | NA | NA | NA | **NA** | **NA** |
| HJ3D11 | -0,5 | -1,7 | **-1,3** | 31,2 | 62,3 | 74,2 | 72,2 | 82,1 | **76,2** | NA | NA | NA | **NA** | 92,0 | 93,6 | 93,5 | **93,0** | **NA** |
| HJ3D12 | -1,2 | -2,2 | **-1,9** | 57,7 | 63,3 | 76,5 | 81,1 | 73,2 | **76,9** | 44,8 | 43,9 | 52,0 | **46,9** | 77,6 | 86,6 | 87,9 | **84,0** | **65,8** |
| HJ3D15 | -0,3 | -1,2 | **-0,9** | 54,7 | 75,0 | 74,1 | 75,8 | 79,6 | **76,5** | NA | NA | NA | **NA** | 78,3 | 79,4 | 87,0 | **81,6** | **NA** |
| HJ3D18 | -0,3 | -1,2 | **-0,9** | 20,8 | 63,8 | 40,3 | 34,8 | 59,0 | **44,7** | NA | NA | NA | **NA** | NA | NA | NA | **NA** | **NA** |
| HJ3D23 | -0,4 | -2,2 | **-1,6** | 44,3 | 65,2 | 80,1 | 81,7 | 80,9 | **80,9** | 41,0 | 37,0 | 45,0 | **41,0** | 90,4 | 97,1 | 94,4 | **94,0** | **69,8** |
| HJ3D38 | 0,0 | -1,8 | **-1,2** | 56,7 | 69,4 | 81,6 | 64,7 | 78,4 | **74,9** | 39,9 | 41,3 | 48,5 | **43,2** | 86,0 | 40,8 | 91,9 | **72,9** | **62,7** |
| HJ3D41 | -0,4 | -1,8 | **-1,3** | 71,3 | 83,1 | 84,2 | 88,3 | 87,3 | **86,6** | 47,5 | 51,3 | 53,5 | **50,8** | 89,7 | 69,2 | 89,1 | **82,7** | **72,6** |
| HMJ3D1 | -0,1 | -1,8 | **-1,2** | 76,1 | 68,6 | 85,5 | 88,3 | 77,7 | **83,8** | 48,6 | 55,6 | 58,4 | **54,2** | 92,1 | 91,1 | 91,1 | **91,4** | **71,8** |

*PC3 = cellules PC3-HsJak3-GFP clone E8

Tableau 4 : Synthèse des expériences réalisées pour tester les siARN dirigés contre JAK3 humain.

**Exemple 2 : Efficacité des siARN.**

a. Contrôle de la spécificité d'inhibition génique :

La spécificité des séquences siARN a été évaluée par des approches de biologie moléculaire, en analysant l'expression génique par RTqPCR des 4 membres de la famille de protéines JAK : JAK1, JAK2, JAK3 et TYK2. Les expériences ont été réalisées avec une concentration de 10 nM de siARN pour favoriser de potentiels effets non-

spécifiques qui sont connus pour être dose-dépendants.

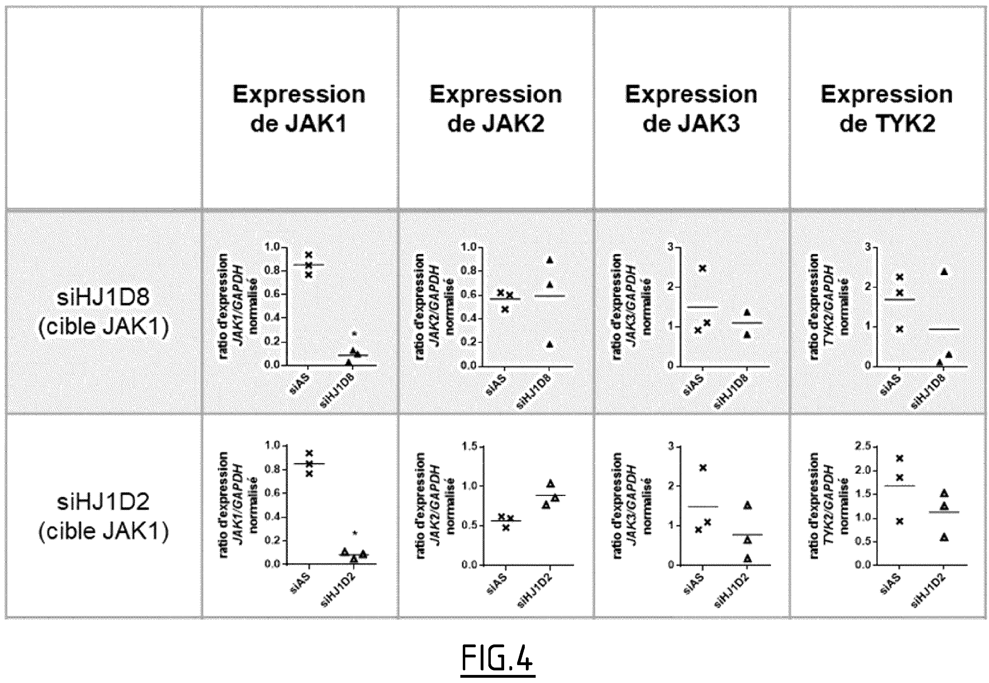

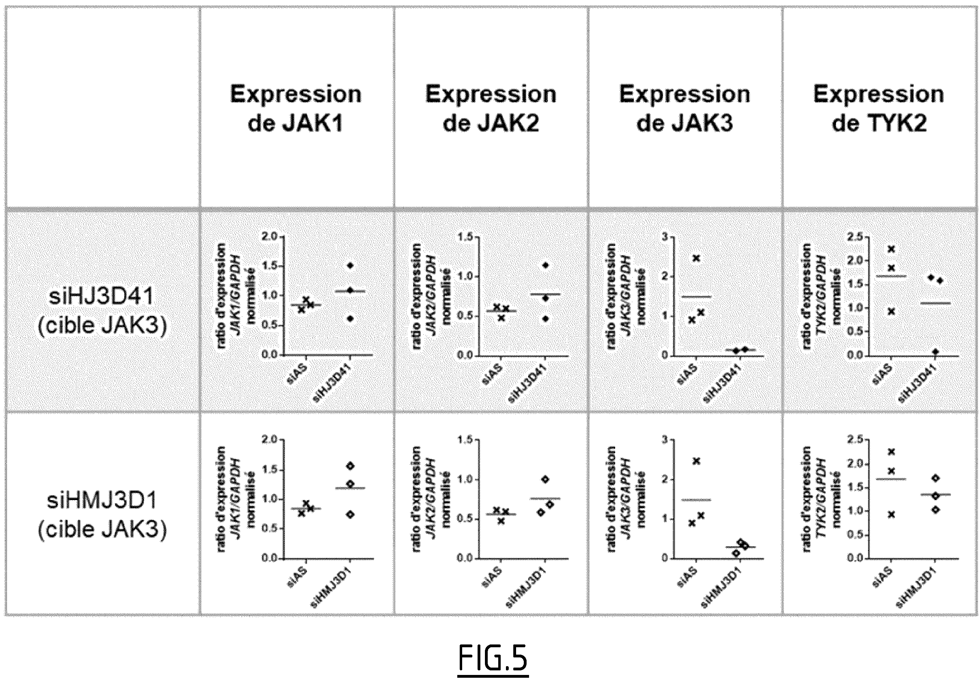

Des cellules Caco-2 ont été transfectées avec 10nM de siARN ciblant JAK1 ou JAK3 en utilisant de la Lipofectamine RNAimax pendant 48h. Les siARN siHJ1D8 et siHJ1D2 ciblent JAK1 et les siHJ3D41 et HMJ3D1 ciblent JAK3. Les ARN ont été extraits, retro-transcrits en ADNc et testés en RTqPCR. Le gène GAPDH a été utilisé comme gène interne et les cellules n'ayant subi aucun traitement ont servi de calibrateur externe. Le siARN « siAS » est un contrôle de transfection qui n'a pas d'homologie pour le génome humain. Les résultats de 3 expériences indépendantes de mesure du niveau d'expression génique JAK1, JAK2, JAK3 ou TYK2 dans les cellules Caco-2 sont montrés en Figures 4 et 5.

Ces résultats ont également été confirmés au niveau protéique par Western-blot. Des cellules PC3 transfectées avec un vecteur pCDNA3.1 dans lequel la séquence codant JAK3 fusionnée à son extrémité C-terminale avec la GFP, ont été transfectées avec 10nM de siARN ciblant JAK1 ou JAK3 en utilisant de la Lipofectamine RNAimax pendant 48h pour permettre le suivi de la protéine JAK3 notamment. Les siARN-JAK impactent uniquement l'expression de leur cible.

b. Contrôle de la sensibilité :

La dose utile de siARN minimale nécessaire pour bloquer l'expression endogène des gènes JAK1 ou JAK3 a été recherchée (si-JAK1 : <12,5 pM dans la lignée humaine épithéliale PC3 ; si-JAK3 : <1 nM dans la lignée humaine épithéliale PC3 surexprimant JAK3 à l'aide de l'introduction d'un plasmide).

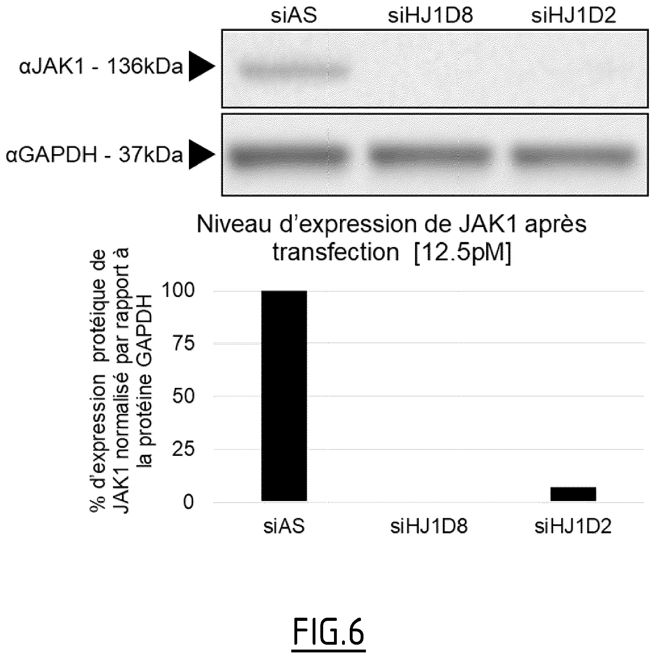

Les cellules PC3 ont été transfectées avec 12,5 pM de siARN en utilisant de la Lipofectamine RNAimax pendant 48h. les cellules ont été lysées en utilisant un tampon de lyse classique. Les lysats protéiques ont ensuite été analysés par Western-blot pour l'expression de JAK1 et de la GAPDH. La Figure 6 représente la quantification de l'expression de JAK1, normalisée vis-à-vis de la GAPDH.

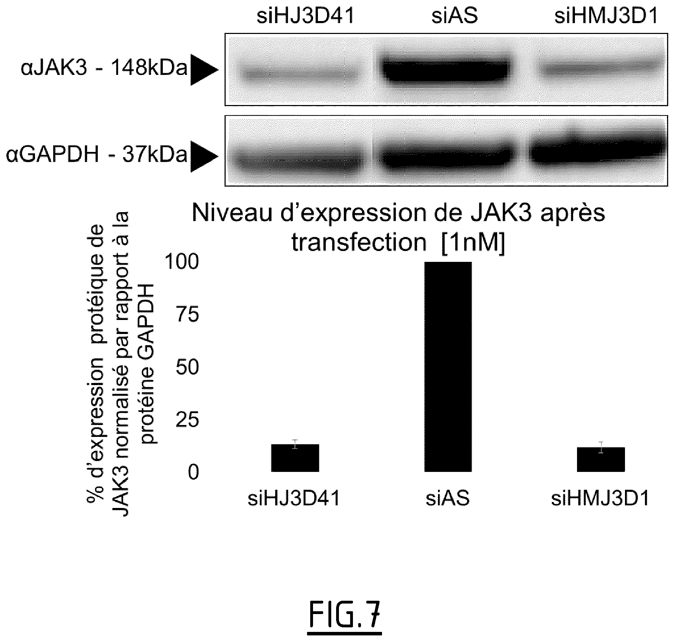

Des cellules PC3 surexprimant JAK3-GFP ont été transfectées avec 1 nM de siARN en utilisant de la Lipofectamine RNAimax pendant 48h. les cellules ont été lysées en utilisant un tampon de lyse classique. Les lysats protéiques ont ensuite été analysés par Western-blot pour l'expression de JAK3 et de la GAPDH. La Figure 7 représente la quantification de l'expression de JAK3.

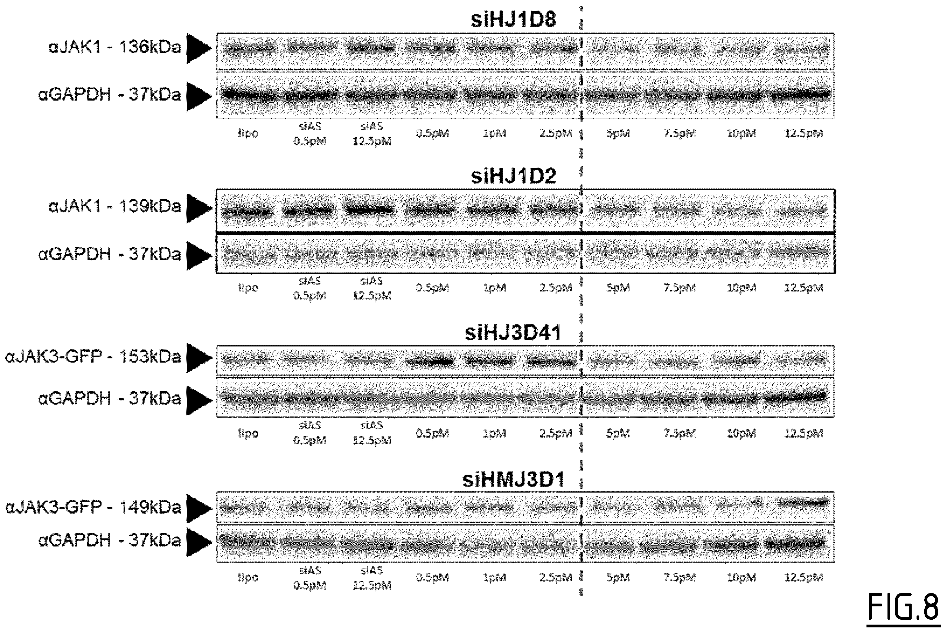

Des cellules PC3 surexprimant JAK3-GFP ont enfin été transfectées avec différentes concentrations de siARN (de 0,5 pM à 12,5 pM) en utilisant de la Lipofectamine RNAimax pendant 48h. les cellules ont été lysées en utilisant un tampon de lyse classique. Les lysats protéiques ont ensuite été analysés par Western-blot pour l'expression de JAK3 ou JAK1 et de la GAPDH. La Figure 8 représente les résultats d'une expérience de Western-blot représentative de 3 expériences indépendantes.

Les séquences siARN proposées restent donc efficaces à des doses bien en dessous de celles préconisées pour les séquences déjà commercialisées (rarement moins de 10 nM). A la concentration de 5 pM, les séquences générées présentent encore une forte efficacité d'inhibition de l'expression de JAK1 ou JAK3 (voir Figure 8).

c. Recherche des effets indésirables :

i. Effets microARN

La présence de séquences nucléotidiques en 3'UTR de l'ensemble du génome humain pouvant être reconnues par les séquences « graines » des siARN a été recherchée.

Tableau 5. Le nombre de fois où la séquence « graine » des si-JAK1 pouvait présenter une complémentarité de séquences avec une séquences nucléotidiques 3'UTR a été recherché *in silico* sur l'ensemble du génome humain.

| | Complémentarité de séquence prédite | | |
|---|---|---|---|
| siARN | 1 hit | 2 hits | >= 3hits |
| HJ1D2 | 339 | 7 | 0 |
| HJ1D8 | 287 | 13 | 0 |

Tableau 6. Le nombre de fois où la séquence « graine » des si-JAK3 pouvait présenter une complémentarité de séquences avec une séquences nucléotidiques 3'UTR a été recherché *in silico* sur l'ensemble du génome humain.

| | Complémentarité de séquence prédite | | |
|---|---|---|---|
| siARN | 1 hit | 2 hits | >= 3hits |
| HJ3D41 | 527 | 8 | 1 |

(suite)

| | Complémentarité de séquence prédite | | |
|---|---|---|---|
| siARN | 1 hit | 2 hits | >= 3hits |
| HMJ3D1 | 1161 | 48 | 3 |

Les résultats obtenus ont été synthétisés dans le Tableau 5 pour les si-JAK1 et dans le Tableau 6 pour les si-JAK3. Les si-JAK sélectionnés présentent un risque très modéré d'activer une réponse microARN.

ii. Effets directs potentiels sur d'autres gènes

L'homologie de séquence des si-JAK pour l'ensemble des gènes humains a été analysée *in silico* à l'aide du programme BLASTN 2.7.0+ (disponible en libre accès) du NCBI. Cette approche a permis d'étudier les homologies de séquences des brins passager et guide des siARN avec l'ensemble du génome humain.

Des cibles potentielles possédant une homologie de séquence partielle avec les siARN ont été mises en évidence.

L'ensemble des gènes possédant une homologie partielle de séquence avec le siHJ1D2 a été compilé dans le Tableau 7.

Tableau 7. Les gènes présentant une homologie de séquence partielle avec les brins passager et guide du siHJ1D2 ont été compilés dans ce tableau. La séquence « graine » du siHJ1D2 est soulignée, l'homologie de séquence entre le gène et le siHJ1D2 est en italique et en gras.

| Nom du gène | % d'homologie | Nucléotides homologues | Positionnement de l'homologie de séquence (séquence « graine » du siHJ1D2 (SEQ ID NO: 6) soulignée) |
|---|---|---|---|
| NPBWR2 | 80% | 17/17 | UU***CGUCAUCCUUGUAAUCC***AU |
| FECH | 71% | 15/15 | UUCG***UCAUCCUUGUAAUCC***AU |
| TAF4 | 71% | 15/15 | U***UCGUCAUCCUUGUAA***UCCAU |
| ADAMTSL2 | 66% | 14/14 | ***UUCGUCAUCCUUGUAA***UCCAU |
| ABLIM1 | 66% | 14/14 | UUCGUC***AUCCUUGUAAUCCA***U |

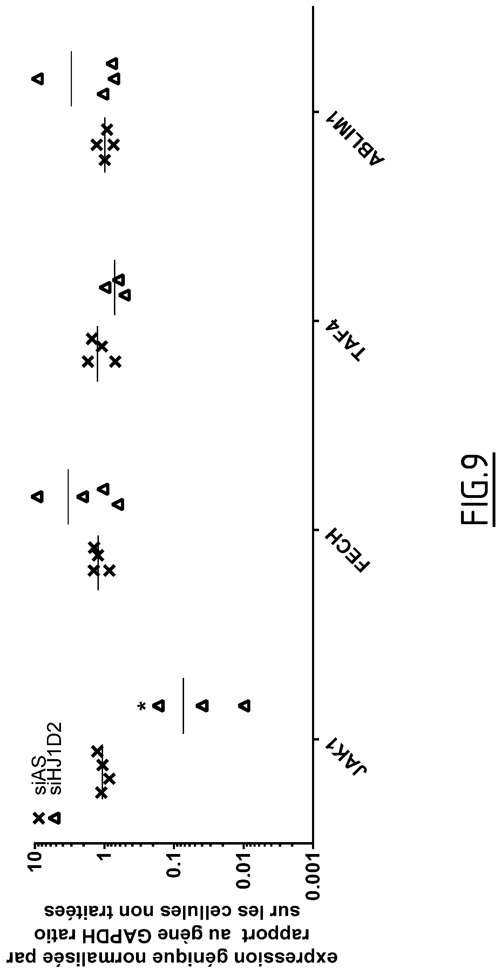

Certaines cibles potentielles trouvées par l'analyse *in silico* ont été validées expérimentalement par RTqPCR. Pour cela, des cellules T47D ont été transfectées avec 10nM de siARN en utilisant de la Lipofectamine RNAimax pendant 48h. Les ARN ont été extraits, retro-transcrits en ADNc et testés en RTqPCR. Le gène GAPDH a été utilisé comme gène interne et les cellules n'ayant subi aucun traitement ont servi de calibrateur externe. Le siARN « siAS » est un contrôle de transfection qui n'a pas d'homologie pour le génome humain. Les résultats montrés en Figure 9 représentent 4 expériences indépendantes en dot plot avec la moyenne en surimpression.

Bien qu'une homologie de séquence partielle entre les gènes FECH, TAF4 et ABLIM1 et le siHJ1D2 ait été détectée *in silico,* ce siARN n'impacte pas l'expression de ces gènes.

L'ensemble des gènes possédant une homologie partielle de séquence avec le siHJ1D8 a été compilé dans le Tableau 8.

Tableau 8. Les gènes présentant une homologie de séquence partielle avec les brins passager et guide du siHJ1D8 ont été compilés dans ce tableau. La séquence « graine » du siHJ1D8 est soulignée, l'homologie de séquence entre le gène et le siHJ1D8 est en italique et en gras.

| Nom du gène | % d'homologie | Nucléotides homologues | Positionnement de l'homologie de séquence (séquence « graine » du siHJ1D8 (SEQ ID NO: 8) soulignée) |
|---|---|---|---|
| ZFYVE1 | 71% | 15/15 | UCGC***UUGUAGCUGAUGUCC***UU |
| ZNF782 | 66% | 14/14 | UCGC***UUGUAGCUGAUGUC***CUU |
| TPMT | 66% | 14/14 | UCGCUUG***UAGCUGAUGUCCUU*** |
| OAS1 | 66% | 14/14 | UCGCUUG***UAGCUGAUGUCCUU*** |

(suite)

| Nom du gène | % d'homologie | Nucléotides homologues | Positionnement de l'homologie de séquence (séquence « graine » du siHJ1D8 (SEQ ID NO: 8) soulignée) |
|---|---|---|---|
| FLO11-like | 61% | 13/13 | UCGCU***UGUAGCUGAUGUC***CUU |
| MFSD14B | 61% | 13/13 | UCGCU***UGUAGCUGAUGUC***CUU |
| BACH2 | 61% | 13/13 | UCG***CUUGUAGCUGAUG***UCCUU |
| CADPS2 | 61% | 13/13 | UCGCU***UGUAGCUGAUGUC***CUU |
| ARL14EP | 61% | 13/13 | ***UCGCUUGUAGCUG***AUGUCCUU |
| NCSTN | 61% | 13/13 | UCGCUUGU***AGCUGAUGUCCUU*** |
| RNH1 | 61% | 13/13 | UCGCUUGU***AGCUGAUGUCCUU*** |
| MEGF10 | 61% | 13/13 | UCGCUUG***UAGCUGAUGUCCU***U |
| FAM160A1 | 76% | 13/13 | UCGCUU***GUAGCUGAUGUCC***UU |
| ZDHHC23 | 61% | 13/13 | UCGC***UUGUAGCUGAUGU***CCUU |

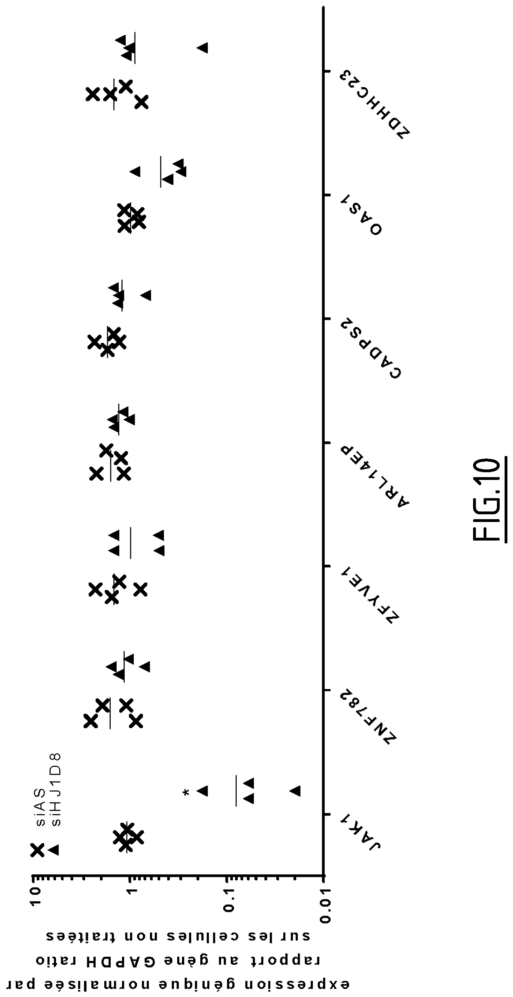

Certaines cibles potentielles trouvées par l'analyse *in silico* ont été validées expérimentalement par RTqPCR. Pour cela des cellules T47D ont été transfectées avec 10nM de siARN en utilisant de la Lipofectamine RNAimax pendant 48h. Les ARN ont été extraits, retro-transcrits en ADNc et testés en RTqPCR. Le gène GAPDH a été utilisé comme gène interne et les cellules n'ayant subi aucun traitement ont servi de calibrateur externe. Le siARN « siAS » est un contrôle de transfection qui n'a pas d'homologie pour le génome humain. Les résultats montrés en Figure 10 représentent 4 expériences indépendantes en dot plot avec la moyenne en surimpression.

Bien qu'une homologie de séquence partielle entre les gènes ZNF782, ZFYVE1, ARL14EP, CADPS2, OAS1 et ZDHHC23 et le siHJ1D8 ait été détectée *in silico,* ce siARN n'impacte pas l'expression de ces gènes.

L'ensemble des gènes possédant une homologie partielle de séquence avec le siHJ3D41 a été compilé dans le Tableau 9.

Tableau 9. Les gènes présentant une homologie de séquence partielle avec les brins passager et guide du siHJ3D41 ont été compilés dans ce tableau. La séquence « graine » du siHJ3D41 est soulignée, l'homologie de séquence entre le gène et le siHJ3D41 est en italique et en gras.

| Nom du gène | % d'homologie | Nucléotides homologues | Positionnement de l'homologie de séquence (séquence « graine » du siHJ3D41 (SEQ ID NO: 2) soulignée) |
|---|---|---|---|
| ZSWIM4 | 76% | 16/16 | UA***CGAUUUCUGGAAAGUC***GCA |
| LRCH2 | 71% | 15/15 | U***ACGAUUUCUGGAAAG***UCGCA |
| RNPEP | 66% | 14/14 | UAC***GAUUUCUGGAAAGU***CGCA |
| PHF21A | 66% | 14/14 | UAC***GAUUUCUGGAAAGU***CGCA |

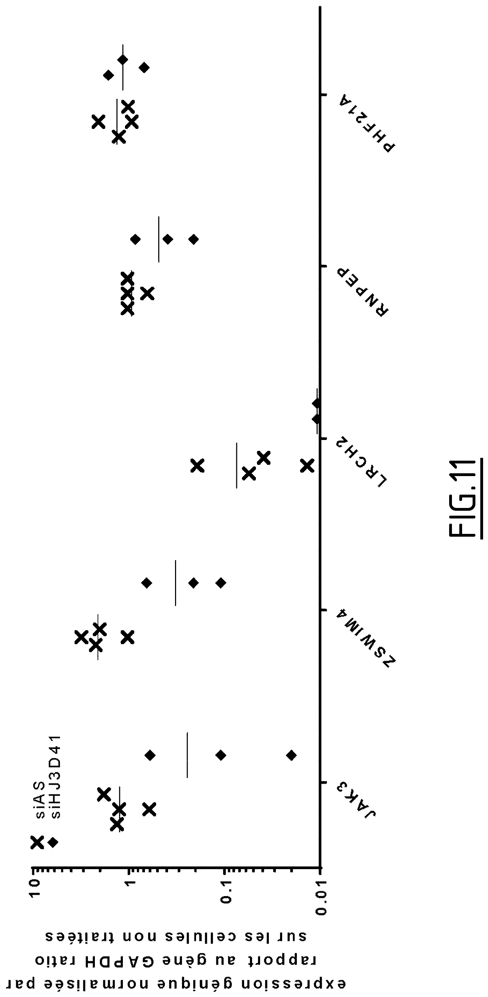

Certaines cibles potentielles trouvées par l'analyse *in silico* ont été validées expérimentalement par RTqPCR. Pour cela des cellules T47D (gènes ZSWIM4, RNPEP et PHF21A) ou PC3 (gène LRCH2 car il n'est pas exprimé par les cellules T47D et ne pouvait donc pas être étudié dans cette lignée) ont été transfectées avec 10nM de siARN en utilisant de la Lipofectamine RNAimax pendant 48h. Les ARN ont été extraits, retro-transcrits en ADNc et testés en RTqPCR. Le gène GAPDH a été utilisé comme gène interne et les cellules n'ayant subi aucun traitement ont servi de calibrateur externe. Le siARN « siAS » est un contrôle de transfection qui n'a pas d'homologie pour le génome humain. Les résultats montrés en Figure 11 représentent 4 expériences indépendantes en dot plot avec la moyenne en surimpression.

Le siHJ3D41 semble diminuer l'expression génique de ZSWIM4 et LRCH2, mais pas celles de RNPEP et PHF21A.

L'ensemble des gènes possédant une homologie partielle de séquence avec le siHMJ3D1 a été compilé dans le Tableau 10.

Tableau 10. Les gènes présentant une homologie de séquence partielle avec les brins passager et guide du siHMJ3D41 ont été compilés dans ce tableau. La séquence « graine » du siHMJ3D41 est soulignée, l'homologie de séquence entre le gène et le siHMJ3D41 est en italique et en gras.

| Nom du gène | % d'homologie | Nucléotides homologues | Positionnement de l'homologie de séquence (séquence « graine » du siHMJ3D1 (SEQ ID NO: 4) soulignée) |
|---|---|---|---|
| KANK3 | 71% | 15/15 | UAG***CGGCACAGCUCCACG***CUG |
| DNAH9 | 66% | 14/14 | UAG***CGGCACAGCUCCAC***GCUG |
| FARP1 | 66% | 14/14 | UAGC***GGCACAGCUCCACG***CUG |
| TMEM120B | 85% | 17/18 | UAGC***GGCACAGCUCCACGCUG*** |
| FAM213A | 66% | 14/14 | UAG***CGGCACAGCUCCAC***GCUG |
| CHD7 | 61% | 13/13 | UAGC***GGCACAGCUCCAC***GCUG |
| TMEM267 | 61% | 13/13 | UA***GCGGCACAGCUCC***ACGCUG |
| MAST4 | 61% | 13/13 | UA***GCGGCACAGCUCCA***CGCUG |
| DGKQ | 61% | 13/13 | UAG***CGGCACAGCUCCA***CGCUG |
| GSDMD | 61% | 13/13 | UAGCGGCA***CAGCUCCACGCUG*** |
| POLDIP3 | 61% | 13/13 | U***AGCGGCACAGCUC***CACGCUG |
| GRIP2 | 61% | 13/13 | UAGCGGCA***CAGCUCCACGCUG*** |
| TEKT4 | 61% | 13/13 | UAG***CGGCACAGCUCCACG***CUG |
| HAUS8 | 61% | 13/13 | UAGC***GGCACAGCUCCACG***CUG |
| YJEFN3 | 61% | 13/13 | UAGCG***GCACAGCUCCACG***CUG |
| FCHO1 | 61% | 13/13 | U***AGCGGCACAGCUC***CACGCUG |
| ELAC2 | 61% | 13/13 | UAG***CGGCACAGCUCCA***CGCUG |
| SGSM3 | 61% | 13/13 | UAGCGGCA***CAGCUCCACGCUG*** |

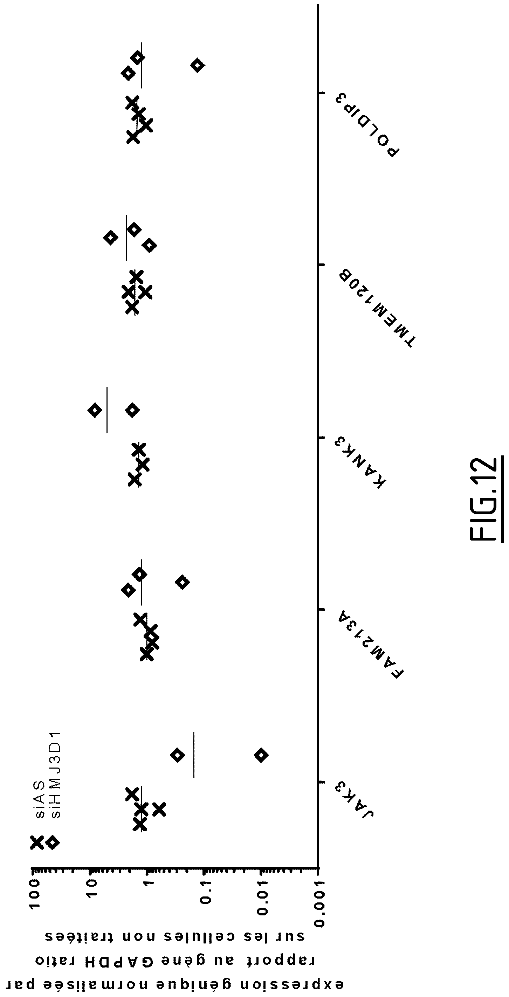

Certaines cibles potentielles trouvées par l'analyse *in silico* ont été validées expérimentalement par RTqPCR. Pour cela, des cellules T47D ont été transfectées avec 10nM de siARN en utilisant de la Lipofectamine RNAimax pendant 48h. Les ARN ont été extraits, retro-transcrits en ADNc et testés en RTqPCR. Le gène GAPDH a été utilisé comme gène interne et les cellules n'ayant subi aucun traitement ont servi de calibrateur externe. Le siARN « siAS » est un contrôle de transfection qui n'a pas d'homologie pour le génome humain. Les résultats montrés en Figure 12 représentent 4 expériences indépendantes en dot plot avec la moyenne en surimpression.

Bien qu'une homologie de séquence partielle entre les gènes FAM213A, KANK3, TMEM120B et POLDIP3 et le siHMJ3D1 ait été détectée *in silico,* ce siARN n'impacte pas l'expression de ces gènes.

iii. Effets phénotypiques

L'impact de l'utilisation des siARN sur les grandes fonctions cellulaires (la prolifération, l'apoptose, et le métabolisme de l'ATP) a été analysé.

Les cellules Caco-2 ont été transfectées avec 10 nM de siARN en utilisant de la Lipofectamine RNAimax.

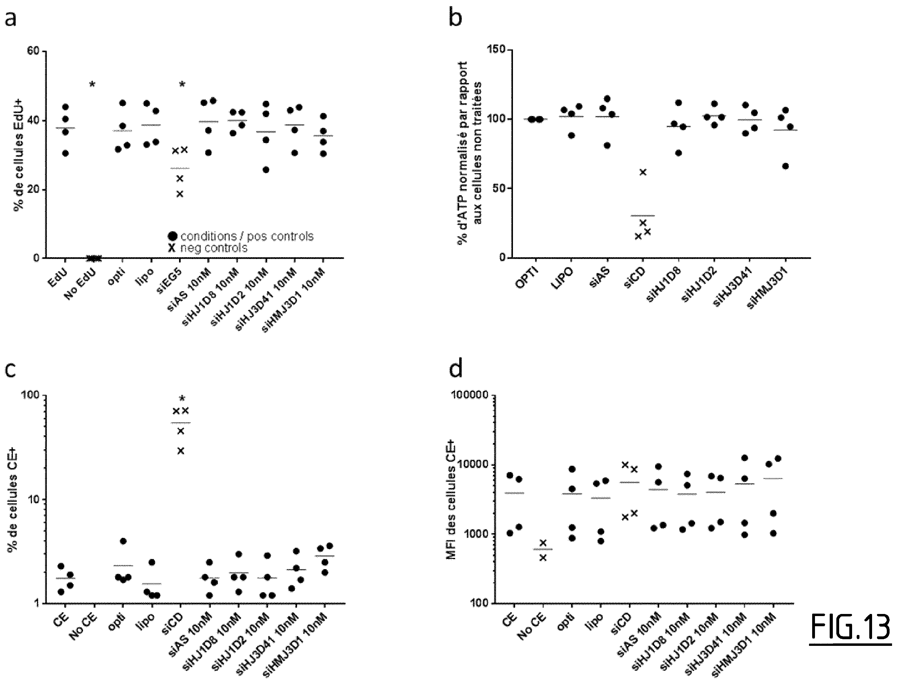

Les cellules transfectées ont ensuite été incubées en présence d'EdU (5μM) pendant 5h, qui est un agent fluorescent qui s'intercale dans l'ADN des cellules qui prolifèrent. Les cellules ont ensuite été décollées puis perméabilisées avant d'être analysées en cytométrie de flux. Le siRNA ciblant le gène EG5 est un contrôle interne d'expérience puisque ce gène est directement impliqué dans la prolifération cellulaire. Les résultats en Figure 13a représentent 4 expériences indépendantes en dot plot avec la moyenne en surimpression, « * » signifie une p-value significative en test statistique apparié non paramétrique. Les siARN d'intérêt n'ont quant à eux aucun impact sur la prolifération.

Par ailleurs, après 48h de transfection, le niveau d'ATP cellulaire a été mesuré à l'aide d'un kit Vialight (Figure 13b.)

Lors de la transfection des cellules ont été mises en présence de CellEvent, un marqueur des capases3/7 activées. Ce marqueur permet de quantifier l'apoptose. Les cellules ont été analysées après perméabilisation en cytométrie de flux. La Figure 13c représente le pourcentage de cellules en apoptose alors que Figure 13d représente

le nombre de molécule fluorescente à la surface des cellules positives.

Les siARN n'ont pas réduit la prolifération cellulaire, ni augmenté l'apoptose ou modifié le métabolisme de l'ATP de manière statistiquement significative.

## Revendications

**1.** Acide ribonucléique (ARN) double brin (db) comprenant un brin sens et un brin antisens, dans lequel :

le brin sens comprend la séquence nucléotidique SEQ ID NO : 7 et le brin antisens comprend la séquence nucléotidique SEQ ID NO : 8,
et chaque brin de l'ARNdb comprend au plus 24 nucléotides,
pour son utilisation comme médicament.

**2.** Acide ribonucléique (ARN) double brin (db) comprenant un brin sens et un brin antisens, dans lequel :

le brin sens comprend la séquence nucléotidique SEQ ID NO : 7 et le brin antisens comprend la séquence nucléotidique SEQ ID NO : 8,
et chaque brin de l'ARNdb comprend au plus 24 nucléotides,
pour son utilisation pour la prévention et/ou le traitement d'une maladie associée à un dysfonctionnement en gain de fonction de la voie de signalisation de la Janus kinase 1 (JAK1), où la maladie est sélectionnée dans le groupe constitué des maladies inflammatoires à médiation immunitaire et des cancers.

**3.** ARNdb pour l'utilisation selon l'une quelconque des revendications 1 à 2, où ledit ARNdb réduit l'expression de la Janus kinase 1 (JAK1).

**4.** ARNdb pour l'utilisation selon l'une quelconque des revendications 1 à 3, pour une utilisation chez un sujet humain.

**5.** Acide ribonucléique (ARN) double brin (db) comprenant un brin sens et un brin antisens, utilisable en thérapie humaine, dans lequel :
le brin sens comprend la séquence nucléotidique SEQ ID NO : 7 et le brin antisens comprend la séquence nucléotidique SEQ ID NO : 8, et chaque brin de l'ARNdb comprend au plus 24 nucléotides.

**6.** Composition pharmaceutique comprenant au moins un ribonucléique (ARN) double brin (db) comprenant un brin sens et un brin antisens dans lequel :

le brin sens comprend la séquence nucléotidique SEQ ID NO : 7 et le brin antisens comprend la séquence nucléotidique SEQ ID NO : 8, et chaque brin de l'ARNdb comprend au plus 24 nucléotides,
et un véhicule pharmaceutiquement acceptable.

**7.** Méthode in vitro pour inhiber l'expression de la Janus kinase 1 (JAK1) dans une cellule, la méthode comprenant :

a. l'introduction dans ladite cellule d'un acide ribonucléique (ARN) double brin (db), ledit ARNdb comprenant un brin sens et un brin antisens dans lequel :
le brin sens comprend la séquence nucléotidique SEQ ID NO : 7 et le brin antisens comprend la séquence nucléotidique SEQ ID NO : 8, et chaque brin de l'ARNdb comprend au plus 24 nucléotides, et
b. le maintien de la cellule produite à l'étape (a) pendant un temps suffisant pour obtenir la dégradation de l'ARNm d'un gène JAK1, inhibant ainsi l'expression de JAK1 dans la cellule.

**8.** Méthode in vitro selon la revendication 7, dans laquelle, à l'étape a, ledit ARNdb est mis en contact avec ladite cellule à une concentration de 5 pM à 10 nM.

**9.** ARNdb pour l'utilisation selon l'une quelconque des revendications 1 à 4, ARNdb selon la revendication 5, composition pharmaceutique selon la revendication 6, ou méthode selon la revendication 7 ou 8, dans lequel ou laquelle le nucléotide à l'extrémité 5' du brin antisens de l'ARNdb est phosphorylé.

**10.** ARNdb pour l'utilisation selon l'une quelconque des revendications 1 à 4 et 9, ARNdb selon la revendication 5 ou 9, composition pharmaceutique selon la revendication 6 ou 9, ou méthode selon l'une quelconque des revendications 7

à 9, dans lequel ou laquelle chaque brin de l'ARNdb comprend au plus 23 ou 22 nucléotides.

**11.** ARNdb pour l'utilisation selon l'une quelconque des revendications 1 à 4 et 9-10, ARNdb selon la revendication 5, 9 ou 10, composition pharmaceutique selon l'une quelconque des revendications 6 et 9 à 10, ou méthode selon l'une quelconque des revendications 7 à 10, dans lequel ou laquelle les deux brins de l'ARNdb sont de longueur identique.

**12.** ARNdb pour l'utilisation selon l'une quelconque des revendications 1 à 4 et 9 à 11, ARNdb selon l'une quelconque des revendications 5 et 9 à 11, composition pharmaceutique selon l'une quelconque des revendications 6 et 9 à 11, ou méthode selon l'une quelconque des revendications 7 à 11, dans lequel ou laquelle l'ARNdb comprend au plus 60 nucléotides.

**Patentansprüche**

**1.** Doppelstrang (db) von Ribonukleinsäure (RNA), umfassend einen Sense-Strang und einen Antisense-Strang, wobei:

der Sense-Strang die Nukleotidsequenz SEQ ID NO: 7 umfasst und der Antisense-Strang die Nukleotidsequenz SEQ ID NO: 8 umfasst, und
jeder Strang der dbRNA höchstens 24 Nukleotiden umfasst,
zur Verwendung als Medikament.

**2.** Doppelstrang (db) von Ribonukleinsäure (RNA), umfassend einen Sense-Strang und einen Antisense-Strang, wobei:

der Sense-Strang die Nukleotidsequenz SEQ ID NO: 7 umfasst und der Antisense-Strang die Nukleotidsequenz SEQ ID NO: 8 umfasst, und
jeder Strang der dbRNA höchstens 24 Nukleotiden umfasst,
zur Verwendung bei der Prävention und/oder Behandlung einer mit einer durch Funktionsgewinn bedingten Fehlfunktion des Signalwegs der mit Januskinase 1 (JAK1) assoziierten Krankheit, wobei diese Krankheit ausgewählt ist aus der Gruppe, bestehend aus immunvermittelten entzündlichen Erkrankungen und Krebserkrankungen.

**3.** dbRNA zur Verwendung nach einem der Ansprüche 1 bis 2, wobei die dbRNA die Expression der Januskinase 1 (JAK1) reduziert.

**4.** dbRNA zur Verwendung nach einem der Ansprüche 1 bis 3 zur Verwendung bei einem menschlichen Subjekt.

**5.** Doppelstrang (db) von Ribonukleinsäure (RNA), umfassend einen Sense-Strang und einen Antisense-Strang, verwendbar in der Humantherapie, wobei:
der Sense-Strang die Nukleotidsequenz SEQ ID NO: 7 umfasst und der Antisense-Strang die Nukleotidsequenz SEQ ID NO: 8 umfasst, undjeder Strang der dbRNA höchstens 24 Nukleotiden umfasst.

**6.** Pharmazeutische Zusammensetzung, umfassend mindestens einen Doppelstrang (db) von Ribonukleinsäure (RNA), umfassend einen Sense-Strang und einen Antisense-Strang, wobei:

der Sense-Strang die Nukleotidsequenz SEQ ID NO: 7 umfasst und der Antisense-Strang die Nukleotidsequenz SEQ ID NO: 8 umfasst, undjeder Strang der dbRNA höchstens 24 Nukleotiden umfasst,
und einen pharmazeutisch annehmbaren Träger.

**7.** *In-vitro-Verfahren* zum Hemmen der Expression der Januskinase 1 (JAK1) in einer Zelle, das Verfahren umfassend:

a. das Einbringen eines Doppelstransg (db) von Ribonukleinsäure (RNA) in die Zelle, die dbRNA umfassend einen Sense-Strang und einen Antisense-Strang, wobei:
der Sense-Strang die Nukleotidsequenz SEQ ID NO: 7 umfasst und der Antisense-Strang die Nukleotidsequenz SEQ ID NO: 8 umfasst, undjeder Strang der dbRNA höchstens 24 Nukleotiden umfasst, und
b. Halten der in Schritt (a) hergestellten Zelle über eine Zeit, die ausreichend ist, um den Abbau der mRNA eines JAK1-Gens zu erlangen, wodurch die Expression von JAK1 in der Zelle gehemmt wird.

**8.** *In-vitro-Verfahren* nach Anspruch 7, wobei die dbRNA in Schritt a in einer Konzentration von 5 pM bis 10 nM mit der

Zelle in Kontakt gebracht wird.

9. dbRNA zur Verwendung nach einem der Ansprüche 1 bis 4, dbRNA nach Anspruch 5, pharmazeutische Zusammensetzung nach Anspruch 6 oder Verfahren nach Anspruch 7 oder 8, wobei oder in dem das Nukleotid an dem 5'-Ende des Antisense-Strangs der dbRNA phosphoryliert ist.

10. dbRNA zur Verwendung nach einem der Ansprüche 1 bis 4 und 9, dbRNA nach Anspruch 5 oder 9, pharmazeutische Zusammensetzung nach Anspruch 6 oder 9 oder Verfahren nach einem der Ansprüche 7 bis 9, wobei oder in dem jeder Strang der dbRNA höchstens 23 oder 22 Nukleotide umfasst.

11. dbRNA zur Verwendung nach einem der Ansprüche 1 bis 4 und 9-10, dbRNA nach Anspruch 5, 9 oder 10, pharmazeutische Zusammensetzung nach einem der Ansprüche 6 und 9 bis 10 oder Verfahren nach einem der Ansprüche 7 bis 10, wobei oder in dem die zwei Stränge der dbRNA von identischer Länge sind.

12. dbRNA zur Verwendung nach einem der Ansprüche 1 bis 4 und 9 bis 11, dbRNA nach einem der Ansprüche 5 und 9 bis 11, pharmazeutische Zusammensetzung nach einem der Ansprüche 6 und 9 bis 11 oder Verfahren nach einem der Ansprüche 7 bis 11, wobei oder in dem die dbRNA höchstens 60 Nukleotide umfasst.

## Claims

1. A double-stranded (ds) ribonucleic acid (RNA) comprising a sense strand and an antisense strand, wherein:

   the sense strand comprises the nucleotide sequence SEQ ID NO: 7 and the antisense strand comprises the nucleotide sequence SEQ ID NO: 8,
   and each strand of the dsRNA comprises at most 24 nucleotides,
   for use thereof as a drug.

2. A double-stranded (ds) ribonucleic acid (RNA) comprising a sense strand and an antisense strand, wherein:

   the sense strand comprises the nucleotide sequence SEQ ID NO: 7 and the antisense strand comprises the nucleotide sequence SEQ ID NO: 8,
   and each strand of the dsRNA comprises at most 24, 23 or 22 nucleotides
   for use thereof in the prevention and/or treatment of a disease associated with a Janus kinase (JAK) gain of function disruption of the Janus kinase 1 (JAK1) signaling pathway, wherein the disease is selected from the group consisting of immune-mediated inflammatory diseases and cancers.

3. A dsRNA for use according to any one of claims 1 to 2, wherein said dsRNA reduces the expression of Janus kinase 1 (JAK1).

4. A dsRNA for use according to any one of claims 1 to 3, for use in a human subject.

5. A double-stranded (ds) ribonucleic acid (RNA) comprising a sense strand and an antisense strand, that is usable in human therapy, wherein:
   the sense strand comprises the nucleotide sequence SEQ ID NO: 7 and the antisense strand comprises the nucleotide sequence SEQ ID NO: 8, and each strand of the dsRNA comprises at most 24 nucleotides.

6. A pharmaceutical composition comprising at least one double-stranded (ds) ribonucleic acid (RNA) comprising a sense strand and an antisense strand wherein:

   the sense strand comprises the nucleotide sequence SEQ ID NO: 7 and the antisense strand comprises the nucleotide sequence SEQ ID NO: 8, and each strand of the dsRNA comprises at most 24 nucleotides,
   and a pharmaceutically acceptable carrier.

7. An *in vitro* method for inhibiting the expression of Janus kinase 1 (JAK1) in a cell, the method comprising:

   a. introduction into said cell of a double-stranded (ds) ribonucleic acid (RNA), said dsRNA comprising a sense strand and an antisense strand wherein:

the sense strand comprises the nucleotide sequence SEQ ID NO: 7 and the antisense strand comprises the nucleotide sequence SEQ ID NO: 8, and each strand of the dsRNA comprises at most 24 nucleotides, and
b. maintaining the cell produced in step (a) for a period of time sufficient to achieve degradation of the mRNA of a JAK1 gene, thereby inhibiting the expression of JAK1 in the cell.

**8.** An *in vitro* method according to claim 7, wherein, in step a, said dsRNA is brought into contact with the said cell at a concentration of 5 pM to 10 nM.

**9.** A dsRNA for use thereof according to any one of claims 1 to 4, a dsRNA according to claim 5, a pharmaceutical composition according to claim 6, or a method according to claims 7 or 8, wherein the nucleotide at the 5' end of the antisense strand of dsRNA is phosphorylated.

**10.** A dsRNA for use thereof according to any one of claims 1 to 4 and 9, a dsRNA according to claims 5 or 9, a pharmaceutical composition according to claims 6 or 9, or a method according to any one of claims 7 to 9; wherein each strand of the dsRNA comprises at most 23 or 22 nucleotides.

**11.** A dsRNA for use thereof according to any one of claims 1 to 4 and 9-10, a dsRNA according to claims 5, 9 or 10, a pharmaceutical composition according to any one of claims 6 and 9 to 10, or a method according to any one of claims 7 to 10, wherein the two strands of the dsRNA are of identical length.

**12.** A dsRNA for use thereof according to any one of claims 1 to 4, and 9 to 11, a dsRNA according to any one of claims 5 and 9 to 11, a pharmaceutical composition according to any one of claims 6 and 9 to 11; or a method according to any one of claims 7 to 11, wherein the dsRNA comprises at most 60 nucleotides.

Brin passager
Brin guide
5'
3'
1 2 3 4 5 6 7 8 9 10 11 12 13 14 15 16 17 18 19 20 21
3'
5'
21 20 19 18 17 16 15 14 13 12 11 10 9 8 7 6 5 4 3 2 1
P
G/C
ratio G/C équilibré
fort taux A/U
A/U


FIG.1

>lcl|NM_002227.3_cds_NP_002218.2_1 [gene=JAK1] [protein=tyrosine-protein kinase JAK1 isoform 1] [protein_id=NP_002218.2] [location=315..3779] Homo Sapiens

MJ1D11 H1D17 HJ1D4

ATGCAGTATCTAAATATAAAAGAGGACTGCAATGCCATGGCTTTCTGTGCTAAAATGAGGAGCTCCAAGAAGACTGAGGTGAACCTGGAGGCCCCTGAGCCAGGGGTGGAAGTGATCTTCTATCTGTCGGACAGGGAGCC

HJ1D64

CCTCCGGCTGGGCAGTGGAGAGTACACAGCAGAGGAACTGTGCATCAGGGCTGCACAGGCATGCCGTATCTCTCCTCTTTGTCACAACCTCTTTGCCCTGTATGACGAGAACACCAAGCTCTGGTATGCTCCAAATCGCA

HJ1D15 HJ1A46 HJ1H12 HJ1D26 HJ1D73

CCATCACCGTTGATGACAAGATGTCCCTCCGGCTCCACTACCGGATGAGGTTCTATTTCACCAATTGGCATGGAACCAACGACAATGAGCAGTCAGTGTGGCGTCATTCTCCAAAGAAGCAGAAAAATGGCTACGAGAAA

HJ1D21 HJ1D13 HJ1H10

AAAAAGATTCCAGATGCAACCCCTCTCCTTGATGCCAGCTCACTGGAGTATCTGTTTGCTCAGGGACAGTATGATTTGGTGAAATGCCTGGCTCCTATTCGAGACCCCAAGACCGAGCAGGATGGACATGATATTGAGAA

HJ1D8 HJ1Q3

CGAGTGTCTAGGGATGGCTGTCCTGGCCATCTCACACTATGCCATGATGAAGAAGATGCAGTTGCCAGAACTGCCCAAGGACATCAGCTACAAGCGATATATTCCAGAAACATTGAATAAGTCCATCAGACAGAGGAACC

MJ1D11

TTCTCACCAGGATGCGGATAAATAATGTTTTCAAGGATTTCCTAAAGGAATTTAACAACAAGACCATTTGTGACAGCAGCGTGTCCACGCATGACCTGAAGGTGAAATACTTGGCTACCTTGGAAACTTTGACAAAACAT

TACGGTGCTGAAATATTTGAGACTTCCATGTTACTGATTTCATCAGAAAATGAGATGAATTGGTTTCATTCGAATGACGGTGGAAACGTTCTCTACTACGAAGTGATGGTGACTGGGAATCTTGGAATCCAGTGGAGGCA

TAAACCAAATGTTGTTTCTGTTGAAAAGGAAAAAAATAAACTGAAGCGGAAAAAACTGGAAAATAAACACAAGAAGGATGAGGAGAAAAACAAGATCCGGGAAGAGTGGAACAATTTTTCTTACTTCCCTGAAATCACTC

HMJ1D1

ACATTGTAATAAAGGAGTCTGTGGTCAGCATTAACAAGCAGGACAACAAGAAAATGGAACTGAAGCTCTCTTCCCACGAGGAGGCCTTGTCCTTTGTGTCCCTGGTAGATGGCTACTTCCGGCTCACAGCAGATGCCCAT

CATTACCTCTGCACCGACGTGGCCCCCCCGTTGATCGTCCACAACATACAGAATGGCTGTCATGGTCCAATCTGTACAGAATACGCCATCAATAAATTGCGGCAAGAAGGAAGCGAGGAGGGGATGTACGTGCTGAGGTG

HMJ1D2

GAGCTGCACCGACTTTGACAACATCCTCATGACCGTCACCTGCTTTGAGAAGTCTGAGCAGGTGCAGGGTGCCCAGAAGCAGTTCAAGAACTTTCAGATCGAGGTGCAGAAGGGCCGCTACAGTCTGCACGGTTCGGACC

HJ1Q6

GCAGCTTCCCCAGCTTGGGAGACCTCATGAGCCACCTCAAGAAGCAGATCCTGCGCACGGATAACATCAGCTTCATGCTAAAACGCTGCTGCCAGCCCAAGCCCCGAGAAATCTCCAACCTGCTGGTGGCTACTAAGAAA

HJ1D2

GCCCAGGAGTGGCAGCCCGTCTACCCCATGAGCCAGCTGAGTTTCGATCGGATCCTCAAGAAGGATCTGGTGCAGGGCGAGCACCTTGGGAGAGGCACGAGAACACACATCTATTCTGGGACCCTGATGGATTACAAGGA

HJ1H11

TGACGAAGGAACTTCTGAAGAGAAGAAGATAAAAGTGATCCTCAAAGTCTTAGACCCCAGCCACAGGGATATTTCCCTGGCCTTCTTCGAGGCAGCCAGCATGATGAGACAGGTCTCCCACAAACACATCGTGTACCTCT

ATGGCGTCTGTGTCCGCGACGTGGAGAATATCATGGTGGAAGAGTTTGTGGAAGGGGGTCCTCTGGATCTCTTCATGCACCGGAAAAGCGATGTCCTTACCACACCATGGAAATTCAAAGTTGCCAAACAGCTGGCCAGT

GCCCTGAGCTACTTGGAGGATAAAGACCTGGTCCATGGAAATGTGTGTACTAAAAACCTCCTCCTGGCCCGTGAGGGCATCGACAGTGAGTGTGGCCCATTCATCAAGCTCAGTGACCCCGGCATCCCCATTACGGTGCT

HJ1A48

GTCTAGGCAAGAATGCATTGAACGAATCCCATGGATTGCTCCTGAGTGTGTTGAGGACTCCAAGAACCTGAGTGTGGCTGCTGACAAGTGGAGCTTTGGAACCACGCTCTGGGAAATCTGCTACAATGGCGAGATCCCCT

TGAAAGACAAGACGCTGATTGAGAAAGAGAGATTCTATGAAAGCCGGTGCAGGCCAGTGACACCATCATGTAAGGAGCTGGCTGACCTCATGACCCGCTGCATGAACTATGACCCCAATCAGAGGCCTTTCTTCCGAGCC

ATCATGAGAGACATTAATAAGCTTGAAGAGCAGAATCCAGATATTGTTTCAGAAAAAAAACCAGCAACTGAAGTGGACCCCACACATTTTGAAAAGCGCTTCCTAAAGAGGATCCGTGACTTGGGAGAGGGCCACTTTGG

HJ1H9

GAAGGTTGAGCTCTGCAGGTATGACCCCGAAGGGGACAATACAGGGGAGCAGGTGGCTGTTAAATCTCTGAAGCCTGAGAGTGGAGGTAACCACATAGCTGATCTGAAAAAGGAAATCGAGATCTTAAGGAACCTCTATC

HJ1A47

ATGAGAACATTGTGAAGTACAAAGGAATCTGCACAGAAGACGGAGGAAATGGTATTAAGCTCATCATGGAATTTCTGCCTTCGGGAAGCCTTAAGGAATATCTTCCAAAGAATAAGAACAAAATAAACCTCAAACAGCAG

CTAAAATATGCCGTTCAGATTTGTAAGGGGATGGACTATTTGGGTTCTCGGCAATACGTTCACCGGGACTTGGCAGCAAGAAATGTCCTTGTTGAGAGTGAACACCAAGTGAAAATTGGAGACTTCGGTTTAACCAAAGC

AATTGAAACCGATAAGGAGTATTACACCGTCAAGGATGACCGGGACAGCCCTGTGTTTTGGTATGCTCCAGAATGTTTAATGCAATCTAAATTTTATATTGCCTCTGACGTCTGGTCTTTTGGAGTCACTCTGCATGAGC

TGCTGACTTACTGTGATTCAGATTCTAGTCCCATGGCTTTGTTCCTGAAAATGATAGGCCCAACCCATGGCCAGATGACAGTCACAAGACTTGTGAATACGTTAAAAGAAGGAAAACGCCTGCCGTGCCCACCTAACTGT

HJ1Q1

CCAGATGAGGTTTATCAACTTATGAGGAAATGCTGGGAATTCCAACCATCCAATCGGACAAGCTTTCAGAACCTTATTGAAGGATTTGAAGCACTTTTAAAATAA HJ1Q12 ->3'UTR

FIG.2

>lcl|NM_000215.3_cds_NP_000206.2_1 [gene=JAK3] [protein=tyrosine-protein kinase JAK3] [protein_id=NP_000206.2] [location=101..3475] Homo Sapiens

ATGGCACCTCCAAGTGAAGAGACGCCCCTGATCCCTCAGCGTTCATGCAGCCTCTTGTCCACGGAGGCTGGTGCCCTGCATGTGCTGCTGCCCGCTCGGGGCCCCGGGCCCCCCAGCGCCTATCTTTCTCCTTTGGGGA

HJ3D23
CCACTTGGCTGAGGACCTGTGCGTGCAGGCTGCCAAGGCCAGCGGCATCCTGCCTGTGTACCACTCCCTCTTTGCTCTGGCCACGGAGGACCTGTCCTGCTGGTTCCCCCCGAGCCACATCTTCTCCGTGGAGGATGCCA

HJ3D15
GCACCCAAGTCCTGCTGTACAGGATTCGCTTTTACTTCCCCAATTGGTTTGGGCTGGAGAAGTGCCACCGCTTCGGGCTACGCAAGGATTTGGCCAGTGCTATCCTTGACCTGCCAGTCCTGGAGCACCTCTTTGCCCAG

CACCGCAGTGACCTGGTGAGTGGGCGCCTCCCCGTGGGCCTCAGTCTCAAGGAGCAGGGTGAGTGTCTCAGCCTGGCCGTGTTGGACCTGGCCCGGATGGCGCGAGAGCAGGCCCAGCGGCCGGGAGAGCTGCTGAAGAC

TGTCAGCTACAAGGCCTGCCTACCCCCAAGCCTGCGCGACCTGATCCAGGGCCTGAGCTTCGTGACGCGGAGGCGTATTCGGAGGACGGTGCGCAGAGCCCTGCGCCGCGTGGCCGCCTGCCAGGCAGACCGGCACTCGC

TCATGGCCAAGTACATCATGGACCTGGAGCGGCTGGATCCAGCCGGGGCCGCCGAGACCTTCCACGTGGGCCTCCCTGGGGCCCTTGGTGGCCACGACGGGCTGGGGCTGCTCCGCGTGGCTGGTGACGGCGGCATCGCC

HJ3D41 HJ3D18 HJ3D8
TGGACCCAGGGAGAACAGGAGGTCCTCCAGCCCTTCTGCGACTTTCCAGAAATCGTAGACATTAGCATCAAGCAGGCCCCGCGCGTTGGCCCGGCCGGAGAGCACCGC*CTGGTCACTGTTACCAGGACAGACAA*CCAGAT

TTTAGAGGCCGAGTTCCCAGGGCTGCCCGAGGCTCTGTCGTTCGTGGCGCTCGTGGACGGCTACTTCCGGCTGACCACGGACTCCCAGCACTTCTTCTGCAAGGAGGTGGCACCGCCGAGGCTGCTGGAGGAAGTGGCCG

HJ3H11
AGCAGTGCCACGGCCCCATCACTCTGGACTTTGCCATCAACAAGCTCAAGACTGGGGGCTCACGTCCTGGCTCCTATGTTCTCCGCCGCAGCCCCCAGGACTTTGACAGCTTCCTCCTCACTGTCTGTGTCCAGAACCCC

CTTGGTCCTGATTATAAGGGCTGCCTCATCCGGCGCAGCCCCACAGGAACCTTCCTTCTGGTTGGCCTCAGCCGACCCCACAGCAGTCTTCGAGAGCTCCTGGCAACCTGCTGGGATGGGGGGCTGCACGTAGATGGGGT

HJ3D38
GGCAGTGACCCTCACTTCCTGCTGTATCCCCAGACCCAAAGAAAAGTCCAACCTGATCGTGGTCCAGAGAGGTCACAGCCCACCCACATCATCCTTGGTTCAGCCCCAATCCCAATACCAGCTGAGTCAGATGACATTTC

HJ3D5 HJ3D12
ACAAGATCCCTGCTGACAGCCTGGAGTGGCATGAGAACCTGGGCCATGGGTCCTTCACCAAGATTTACCGGGGCTGTCGCCATGAGGTGGTGGATGGGGAGGCCCGAAAGACAGAGGTGCTGCTGAAGGTCATGGATGCC

AAGCACAAGAACTGCATGGAGTCATTCCTGGAAGCAGCGAGCTTGATGAGCCAAGTGTCGTACCGGCATCTCGTGCTGCTCCACGGCGTGTGCATGGCTGGAGACAGCACCATGGTGCAGGAATTTGTACACCTGGGGGC

HJ3D11
CATAGACATGTATCTGCGAAAACGTGGCCACCTGGTGCCAGCCAGCTGGAAGCTGCAGGTGGTCAAACAGCTGGCCTACGCCCTCAACTATCTGGAGGACAAAGGCCTGCCCCATGGCAATGTCTCTGCCCGGAAGGTGC

HJ3Q6
TCCTGGCTCGGGAGGGGGCTGATGGGAGCCCGCCCTTCATCAAGCTGAGTGACCCTGGGGTCAGCCCCGCTGTGTTAAGCCTGGAGATGCTCACCGACAGGATCCCCTGGGTGGCCCCCGAGTGTCTCCGGGAGGCGCAG

HJ3H10 HJ3A52
ACACTTAGCTTGGAAGCTGACAAGTGGGGCTTCGGCGCCACGGTCTGGGAAGTGTTTAGTGGCGTCACCATGCCCATCAGTGCCCTGGATCCTGCTAAGAAACTCCAATTTTATGAGGACCGGCAGCAGCTGCCGGCCCC

HJ3H9
CAAGTGGACAGAGCTGGCCCTGCTGATTCAACAGTGCATGGCCTATGAGCCGGTCCAGAGGCCCTCCTTCCGAGCCGTCATTCGTGACCTCAATAGCCTCATCTCTTCAGACTATGAGCTCCTCTCAGACCCCACACCTG

HMJ3D1
GTGCCCTGGCACCTCGTGATGGGCTGTGGAATGGTGCCCAGCTCTATGCCTGCCAAGACCCCACGATCTTCGAGGAGAGACACCTCAAGTACATCTCACAGCTGGGCAAGGGCAACTTTGGCAGCGTGGAGCTGTGCCGC

HJ3Q1 HJ3D1 HJ3A54
TATGACCCGCTAGGCGACAATACAGGTGCCCTGGTGGCCGTGAAACAGCTGCAGCACAGCGGGCCAGACCAGCAGAGGGACTTTCAGCGGGAGATTCAGATCCTCAAAGCACTGCACAGTGATTTCATTGTCAAGTATCG

HJ3S3
TGGTGTCAGCTATGGCCCGGGCCGCCAGAGCCTGCGGCTGGTCATGGAGTACCTGCCCAGCGGCTGCTTGCGCGACTTCCTGCAGCGGCACCGCGCGCGCCTCGATGCCAGCCGCCTCCTTCTCTATTCCTCGCAGATCT

GCAAGGGCATGGAGTACCTGGGCTCCCGCCGCTGCGTGCACCGCGACCTGGCCGCCCGAAACATCCTCGTGGAGAGCGAGGCACACGTCAAGATCGCTGACTTCGGCCTAGCTAAGCTGCTGCCGCTTGACAAAGACTAC

HJ3H12
TACGTGGTCCGCGAGCCAGGCCAGAGCCCCATTTTCTGGTATGCCCCCGAATCCCTCTCGGACAACATCTTCTCTCGCCAGTCAGACGTCTGGAGCTTCGGGGTCGTCCTGTACGAGCTCTTCACCTACTGCGACAAAAG

CTGCAGCCCCTCGGCCGAGTTCCTGCGGATGATGGGATGTGAGCGGGATGTCCCCGCCCTCTGCCGCCTCTTGGAACTGCTGGAGGAGGGCCAGAGGCTGCCGGCGCCTCCTGCCTGCCCTGCTGAGGTTCACGAGCTCA

TGAAGCTGTGCTGGGCCCCTAGCCCACAGGACCGGCCATCATTCAGCGCCCTGGGCCCCCAGCTGGACATGCTGTGGAGCGGAAGCCGGGGGTGTGAGACTCATGCCTTCACTGCTCACCCAGAGGGCAAACACCACTCC

CTGTCCTTTTCATAG

HJ3Q2 et HJ3Q11 -> 3'UTR

FIG.3

| | Expression de JAK1 | Expression de JAK2 | Expression de JAK3 | Expression de TYK2 |
|---|---|---|---|---|
| siHJ1D8 (cible JAK1) | ratio d'expression *JAK1/GAPDH* normalisé; 0.0 0.2 0.4 0.6 0.8 1.0; siAS siHJ1D8; * | ratio d'expression *JAK2/GAPDH* normalisé; 0.0 0.2 0.4 0.6 0.8 1.0; siAS siHJ1D8 | ratio d'expression *JAK3/GAPDH* normalisé; 0 1 2 3; siAS siHJ1D8 | ratio d'expression *TYK2/GAPDH* normalisé; 0 1 2 3; siAS siHJ1D8 |
| siHJ1D2 (cible JAK1) | ratio d'expression *JAK1/GAPDH* normalisé; 0.0 0.2 0.4 0.6 0.8 1.0; siAS siHJ1D2; * | ratio d'expression *JAK2/GAPDH* normalisé; 0.0 0.5 1.0 1.5; siAS siHJ1D2 | ratio d'expression *JAK3/GAPDH* normalisé; 0 1 2 3; siAS siHJ1D2 | ratio d'expression *TYK2/GAPDH* normalisé; 0.0 0.5 1.0 1.5 2.0 2.5; siAS siHJ1D2 |

FIG.4

Expression de JAK1
Expression de JAK2
Expression de JAK3
Expression de TYK2
siHJ3D41 (cible JAK3)
siHMJ3D1 (cible JAK3)
ratio d'expression JAK1/GAPDH normalisé
ratio d'expression JAK2/GAPDH normalisé
ratio d'expression JAK3/GAPDH normalisé
ratio d'expression TYK2/GAPDH normalisé
siAS
siHJ3D41
siHMJ3D1


FIG.5

siAS
siHJ1D8
siHJ1D2
αJAK1 - 136kDa
αGAPDH - 37kDa
Niveau d'expression de JAK1 après transfection [12.5pM]
% d'expression protéique de JAK1 normalisé par rapport à la protéine GAPDH
100
75
50
25
0
siAS
siHJ1D8
siHJ1D2

FIG.6

siHJ3D41
siAS
siHMJ3D1
αJAK3 - 148kDa
αGAPDH - 37kDa
Niveau d'expression de JAK3 après transfection [1nM]
% d'expression protéique de JAK3 normalisé par rapport à la protéine GAPDH
100
75
50
25
0
siHJ3D41
siAS
siHMJ3D1

FIG.7

FIG.8
siHJ1D8
αJAK1 - 136kDa
αGAPDH - 37kDa
siHJ1D2
αJAK1 - 139kDa
αGAPDH - 37kDa
siHJ3D41
αJAK3-GFP - 153kDa
αGAPDH - 37kDa
siHMJ3D1
αJAK3-GFP - 149kDa
αGAPDH - 37kDa
lipo
siAS 0.5pM
siAS 12.5pM
0.5pM
1pM
2.5pM
5pM
7.5pM
10pM
12.5pM

expression génique normalisée par
rapport au gène GAPDH ratio
sur les cellules non traitées
10
1
0.1
0.01
0.001
siAS
siHJ1D2
*
JAK1
FECH
TAF4
ABLIM1

FIG.9

siAS
siHJ1D8
expression génique normalisée par rapport au gène GAPDH ratio sur les cellules non traitées
10
1
0.1
0.01
*
JAK1
ZNF782
ZFYVE1
ARL14EP
CADPS2
OAS1
ZDHHC23


FIG.10

siAS
siHJ3D41
10
1
0.1
0.01
expression génique normalisée par rapport au gène GAPDH ratio sur les cellules non traitées
JAK3
ZSWIM4
LRCH2
RNPEP
PHF21A

FIG.11

expression génique normalisée par
rapport au gène GAPDH ratio
sur les cellules non traitées
100
10
1
0.1
0.01
0.001
siAS
siHMJ3D1
JAK3
FAM213A
KANK3
TMEM120B
POLDIP3

FIG.12

a
% de cellules EdU+
60
40
20
0
conditions / pos controls
neg controls
EdU
No EdU
opti
lipo
siEG5
siAS 10nM
siHJ1D8 10nM
siHJ1D2 10nM
siHJ3D41 10nM
siHMJ3D1 10nM
b
% d'ATP normalisé par rapport aux cellules non traitées
150
100
50
0
OPTI
LIPO
siAS
siCD
siHJ1D8
siHJ1D2
siHJ3D41
siHMJ3D1
c
% de cellules CE+
100
10
1
CE
No CE
opti
lipo
siCD
siAS 10nM
siHJ1D8 10nM
siHJ1D2 10nM
siHJ3D41 10nM
siHMJ3D1 10nM
d
MFI des cellules CE+
100000
10000
1000
100
CE
No CE
opti
lipo
siCD
siAS 10nM
siHJ1D8 10nM
siHJ1D2 10nM
siHJ3D41 10nM
siHMJ3D1 10nM

FIG.13